(19) Europäisches Patentamt
European Patent Office
Office européen des brevets

(11) EP 0 559 700 B1

(12) **EUROPEAN PATENT SPECIFICATION**

(45) Date of publication and mention of the grant of the patent:
**26.04.2000 Bulletin 2000/17**

(21) Application number: **91920512.0**

(22) Date of filing: **27.11.1991**

(51) Int. Cl.$^7$: **C07D 233/64**, C07D 405/14, A61K 31/415

(86) International application number:
**PCT/FI91/00359**

(87) International publication number:
**WO 92/09583 (11.06.1992 Gazette 1992/13)**

(54) **NOVEL PILOCARPINE DERIVATIVES AND PROCESS FOR THEIR PREPARATION**
NEUE PILOCARPINDERIVATE UND VERFAHREN ZU IHRER HERSTELLUNG
NOUVEAUX DERIVES DE PILOCARPINE ET LEUR PROCEDE DE PREPARATION

(84) Designated Contracting States:
**AT BE CH DE DK ES FR GB GR IT LI LU NL SE**

(30) Priority: **30.11.1990 FI 905930**

(43) Date of publication of application:
**15.09.1993 Bulletin 1993/37**

(73) Proprietor: **Santen Oy**
**33720 Tampere (FI)**

(72) Inventors:
- **JÄRVINEN, Tomi**
  **SF-70200 Kuopio (FI)**
- **PEURA, Pekka**
  **SF-70200 Kuopio (FI)**
- **SUHONEN, Pekka**
  **SF-70200 Kuopio (FI)**
- **URTTI, Arto**
  **SF-70420 Kuopio (FI)**
- **HANHIJÄRVI, Hannu**
  **SF-20600 Turku (FI)**
- **POHJALA, Esko**
  **SF-33720 Tampere (FI)**

(74) Representative:
**Karvinen, Leena Maria et al**
**Oy Jalo Ant-Wuorinen Ab**
**Iso Roobertinkatu 4-6 A**
**00120 Helsinki (FI)**

(56) References cited:
**EP-A- 0 106 541**

- **International Journal of Pharmaceutics, Vol. 75, 1991 TOMI JAERVINEN et al: "0,0'(1,4-Xylylene)bispilocarpic acid esters as new potential double produgs of pilocarpine for improved ocular delivery. I. Synthesis and analysis", see page 249 - page 258.**
- **International Journal of Pharmaceutics, Vol. 75, 1991 TOMI JAERVINEN et al: "0,0'-(1,4-Xylylene) bispilocarpic acid esters as new potentialdouble produgs of pilocarpine for improved ocular delivery. II. Physicochemical properties, stability, solubility and enzymatic hydrolysis", see page 259 - page 269.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 559 700 B1

## Description

The present invention relates to novel pilocarpine prodrug compounds useful for the treatment of glaucoma, and specifically to bispilocarpic acid esters, processes for the preparation of the said novel compounds, pharmaceutical compositions containing the novel compounds and their use.

(+)-pilocarpine, (3S-cis)-3-ethyl-dihydro-4-[(1-methyl-1H-imidazol-5-yl) methyl]-2(3H)-furanone, is a drug which is used for the treatment of glaucoma, which lowers the ocular pressure by increasing the flow of chamber fluid from the eye. The intraocular pressure reducing effect of pilocarpine is based on the ciliary muscle contracting effect of the drug widening the angle of the anterior chamber which is important from the viewpoint of the outflow of the chamber fluid and the outflow of the fluid is facilitated.

The reduction of the intraocular pressure is, however, not the only effect of pilocarpine in the eye. When the drug concentration is sufficiently high, the contracting effect of pilocarpine on the ciliary muscle is increased, resulting in the adaptation of the ocular lens for seeing at close distance. It is then difficult for the patient to accommodate the eye for seeing at a greater distance, which is inconvenient for the patient. Pilocarpine also causes the iris of the eye to contract, the pupil of the eye decreasing considerably. Besides these effects on the eye which are unnecessary from a medical point of view and unpleasant for the patient, pilocarpine may cause side effects outside the eye. Such effects are i.a. increased salivation and bradycardia.

Conventionally glaucoma patients administer pilocarpine locally as eyedrops. Administered in such a manner, however, only about 1 % of the pilocarpine dose is absorbed by the eye and about 70 % in the blood stream. The low absorption rate of pilocarpine in the eye is due to three major factors:

1) the drop is quickly flushed away from the surface of the eye
2) the rapid absorbance of pilocarpine into the blood stream through the conjunctiva of the inner surface of the eyelid
3) the poor corneal penetration capability of pilocarpine.

Pilocarpine is absorbed into the eye through the cornea. In the cornea it is first absorbed in the dense epithelium layer on the eye surface containing cell membrane lipids (fats) in abundance. However, pilocarpine is not very fat soluble wherefore it penetrates relatively little into the corneal epithelium. The corneal epithelium functions simultaneously as a film restricting the absorption of pilocarpine, and as a storage, which delivers pilocarpine through the aqueous stroma and endothelium of the cornea into the fluid of the anterior chamber. From the chamber fluid pilocarpine has easy access to its action site, the ciliary muscle. The duration of the effect of pilocarpine in the eye is substantially reduced by its partial conversion to inactive pilocarpic acid and the rapid departure of pilocarpine from the eye through the chamber fluid circulation and the blood circulation of the iris.

The low absorption into the inner parts of the eye and the short duration of action of pilocarpine administered into the eye cause difficulties in drug treatment. In order to improve the action of the drug and increase its duration of action, pilocarpine must be used in relatively big doses. From this follows that high pilocarpine levels are obtained in the chamber fluid, in the iris and the ciliary muscle which lead to a strong contraction of the pupil and adaptation of the eye to seeing at close distance. Increasing the dose of pilocarpine is, in addition, a relatively ineffective way of prolonging the action of the drug, the drug being of the type that is rapidly excreted from the eye, and thus pilocarpine eyedrops are administered 3 to 8 times daily depending on the patient. Administration of eyedrops so frequently is inconvenient from the point of view of the patient, especially when the administration of the drops is always followed by side effects in the eye. The use of big doses also increase the amount of pilocarpine absorbed in the blood circulation and thus also the risk for other side effects.

Efforts have been aimed at solving the afore said disadvantages relating to the poor absorption of pilocarpine by using pilocarpine prodrug derivatives which absorb better into the corneal epithelium. Such derivatives have to be more fat soluble than pilocarpine in order to improve absorption. In addition, they have to degrade as completely as possible in the corneal epithelium to liberate the pharmaceutically effective pilocarpine and the ineffective pro-moiety. The degree of degradation in the cornea is dependant on the residence time of the derivative in the corneal epithelium and its degradation rate therein. The residence time of the derivative in the epithelium is increased with increased lipophilicity and decreased diffusion coefficient.

Up to now two kinds of prodrug derivatives of pilocarpine have been developed. Bodor discloses in the US-patent 4,061,722 pilocarpine prodrugs based on quaternary ammonium compounds. Bundgaard et al have disclosed in EP-patent application 0 106 541 pilocarpic acid diesters, by means of which improved ocular absorption has been reached. The said pilocarpic diesters are, however, associated with certain disadvantages, such as incomplete degradation in the cornea, as a result of which intact prodrug reaches the chamber fluid, thus leading to little advantage obtainable with the prodrug. Also, a great number of undesirable side products are released from the diesters as compared to the active agent itself, pilocarpine.

The present invention relates to novel bispilocarpic acid esters, i.e. bispilocarpates, by means of which the aforementioned disadvantages may largely be eliminated or at least minimized. Thus the prodrug derivatives according to the invention degrade at least as rapidly to pilocarpine and the pro-moiety when compared to the prodrugs of Bundgaard et al of corresponding lipophilicity, and they also promote at least to the same degree the penetration of pilocarpine through the cornea. In addition, the bispilocarpate derivatives carry into the cornea one pro-moiety for every two pilocarpine molecules, whereas the derivatives of Bundgaard et al carry one pro-moiety for every pilocarpine molecule. The diffusion coefficients of the bispilocarpate derivatives in the corneal epithelium is smaller than those of the Bundgaard compounds, wherefore still undegraded bispilocarpate derivatives remain in the corneal epithelium longer. Thus there will be more time left for the prodrug to break down completely. In addition, the novel compounds of the invention have a better solubility, and are thus better suited for the preparation of drug formulations.

Thus the invention allows for extended slow drug release from the cornea into the inner parts of the eye, by means of which it is possible to effectively prolong the duration of action of pilocarpine and also reduce the peaks of high pilocarpine concentration in the eye, which is of importance from the view of reducing the afore mentioned side-effects.

The novel pilocarpine prodrug-derivatives are thus dimeric ester derivatives of pilocarpic acid, i.e. bispilocarpates of which there are three major types in accordance with the following subgroups A) to C). The bispilocarpates of the subgroup A) may be illustrated with the formula I'

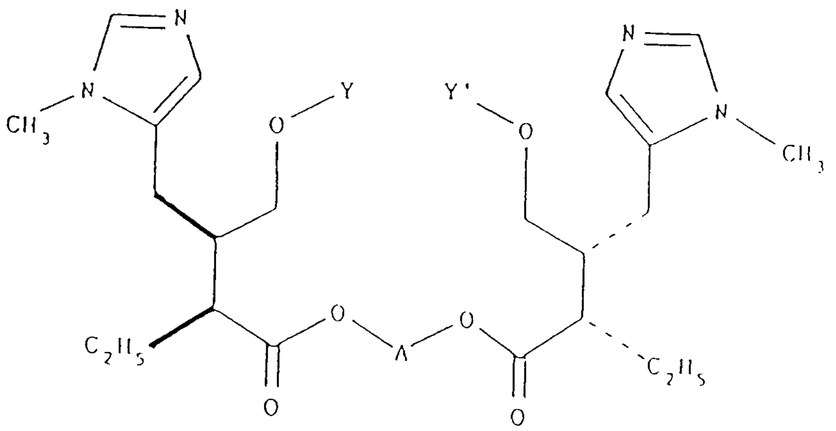

(I')

wherein

Y is hydrogen or

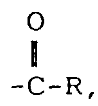

wherein R is hydrogen, $C_1$-$C_{18}$-alkyl, $C_2$-$C_{18}$-alkenyl, $C_2$-$C_{18}$-alkynyl, unsubstituted or $C_1$-$C_4$-alkyl substituted $C_3$-$C_7$-cycloalkyl or $C_3$-$C_7$-cycloalkenyl, unsubstituted or $C_1$-$C_4$-alkyl, $C_1$-$C_4$-alkoxy, nitro or halogen substituted aryl or aryl lower alkyl with up to 6 carbons in the alkyl moiety, and

Y' has the meaning of hydrogen or the group

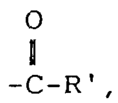

wherein R' has the meaning of R above, whereby R' can be the same as or different from R, A is optionally hydroxy or protected-hydroxy substituted $C_1$-$C_{18}$-alkylene, $C_2$-$C_{18}$-alkenylene or $C_2$-$C_{18}$-alkynylene, which may be substituted by unsubstituted or $C_1$-$C_4$-alkyl substituted $C_3$-$C_7$-cycloalkyl, $C_3$-$C_7$-cycloalkenyl, unsubstituted or $C_1$-$C_4$-alkyl, $C_1$-$C_4$-alkoxy, nitro or halogen substituted aryl or aryl lower alkyl, with up to 6 carbons in the alkyl moiety, or

A is unsubstituted or $C_1$-$C_4$-alkyl substituted $C_3$-$C_7$-cycloalkylene or $C_3$-$C_7$-cycloalkenylene, or unsubstituted or $C_1$-$C_4$-alkyl, $C_1$-$C_4$-alkoxy, nitro or halogen substituted arylene, or A is the aforementioned alkylene, alkenylene, or alkynylene, which as a chain member contains the afore defined cycloalkylene, cycloalkenylene or arylene group.

The bispilocarpates of the subgroup B) may be illustrated with the formula I"

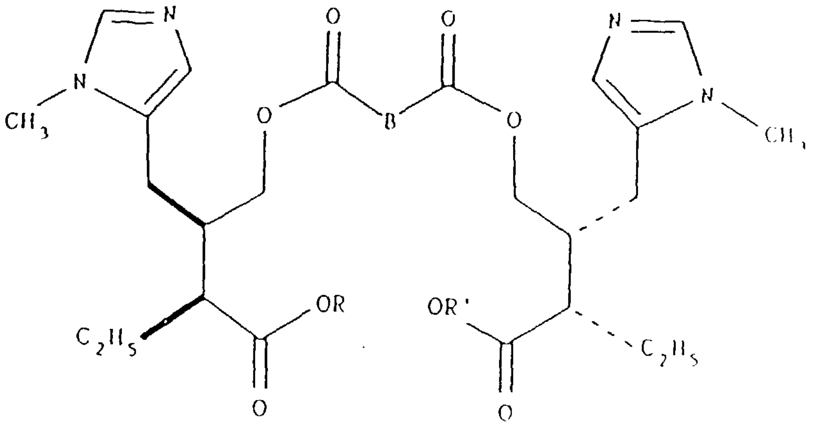

(I")

wherein R has the meaning given above, R' has the meaning given above and B has the meaning given for A above.

The bispilocarpate macrolides in accordance with the subgroup c) may be illustrated with the formula I'"

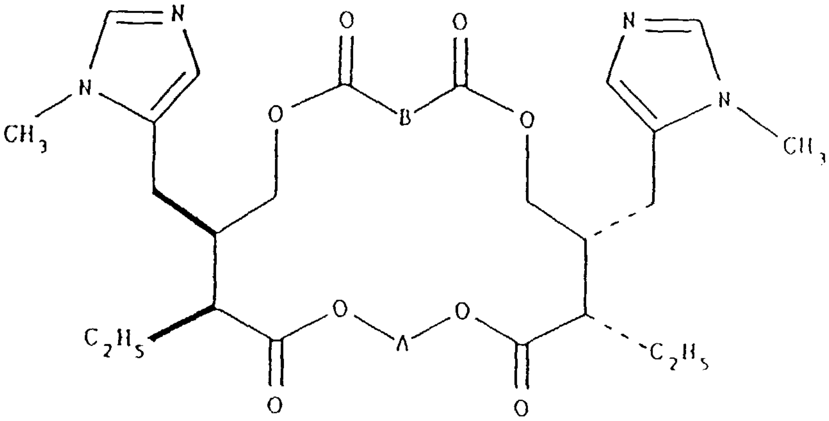

(I"')

wherein A and B have the meaning given above, as well as the acid addition salts of the said compounds.

In connection with the afore mentioned general formulae I', I" and I'" $C_1$-$C_{18}$-alkyl is straight or branched, advantageously lower alkyl with 1-10 C-atoms, preferably 1-4 C-atoms, such as methyl, ethyl, propyl, isopropyl or butyl, isobutyl, sec-butyl or tert-butyl, or pentyl, hexyl, heptyl, octyl, nonyl and decyl.

$C_2$-$C_{18}$-alkenyl may be straight or branched and is advantageously lower alkenyl with 2-10 C-atoms, preferably 2-4 C-atoms and means ethenyl, 1-methylethenyl, 1-propenyl, allyl or 1-, 2- or 3-butenyl, 2-methyl-2-propenyl or also 1-, 2-, 3- or 4-pentenyl, iso-pentenyl, 3-methyl-2-butenyl, hexenyl, heptenyl, octenyl, nonenyl or decenyl. The said alkenyls may be either in E-or Z-form, or may be conjugated or non-conjugated dienyls, such as 3,7-dimethyl-2,6-octadiene, trienyls, such as farnesyl, or polyenyls.

$C_2$-$C_{18}$-alkynyl may be straight or branched and is advantageously lower alkynyl with 2-10 C-atoms, preferably 2-4 C-atoms, and means for example ethynyl, 1-propynyl, propargyl or butynyl, or also pentynyl, hexynyl, heptynyl, octynyl, nonynyl or decynyl. Also conjugated or non-conjugated di-, tri- and poly-ynyls and alkenynyls come into question.

Cycloalkyl and -alkenyl, and cycloalkylene and -alkenylene, respectively, contain 3-7 C-atoms and may be unsubstituted or substituted with lower alkyl, wherein lower alkyl preferably contains 1-4 C-atoms.

Aryl means a substituted or unsubstituted carbocyclic aromatic ring, such as phenyl, or a bicyclic unsaturated or partly saturated ring system, such as for example naphtyl, indenyl, indanyl, tetrahydronaphtyl or biphenyl, etc. As substituents lower alkyl, lower alkoxy, nitro or halogen come into question, whereby lower alkyl and lower alkoxy preferably contain 1-4 C-atoms.

Monocyclic aryl and aralkyl may be illustrated with the formula

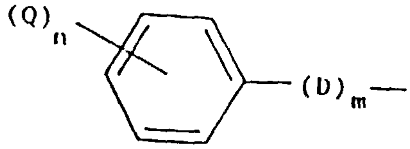

wherein the groups Q independently have the meaning of $C_1$-$C_4$-alkyl, $C_1$-$C_4$-alkoxy, halogen or nitro, n is an integer 0-3, m is an integer 0 or 1, and D means a straight or branched $C_1$-$C_6$-alkylene or conjugated or non-conjugated $C_2$-$C_6$-alkenylene or -alkynylene. Halogen is chlorine, bromine, fluorine or iodine.

A means as a bivalent alkylene, alkenylene, or alkynylene group such a straight or branched bivalent group which contains 1-18 or 2-18 C-atoms, respectively, preferably however 1-10 and 2-10 C-atoms, respectively, such as methylene, ethylene, propylene, butylene, but also pentylene, hexylene, heptylene, octylene, nonylene, and decylene, and corresponding unsaturated bivalent groups, which as a chain substituent may contain the afore defined cycloalkyl, cycloalkenyl, aryl or aralkyl group, or which may be substituted by hydroxy or protected-hydroxy. Protected-hydroxy is the group -OR'' or -OCOR'', wherein R'' means $C_1$-$C_4$-alkyl, aryl or aryl-$C_1$-$C_4$-alkyl, wherein aryl has the meaning given above.

A may as cycloalkylene and cycloalkenylene, respectively, mean for example cyclopropylene, cyclobutylene, cyclopentylene, cyclohexylene, or cycloheptylene, and the corresponding unsaturated groups. The last mentioned bivalent ring systems may also form part of the said alkylene, alkenylene and alkynylene groups.

Arylene as such or as a member of a chain may be illustrated with the following formula

$$-(D)_m-Ar-(D')_{m'}-$$

wherein the bivalent Ar has the meaning of the above defined optionally substituted aryl group, D and D' are the same and different and have the meaning of D above, especially, methylene or ethylene, and m and m' are independently integers 0 or 1. Arylene is preferably phenylene or naphtylene.

Acid addition salts of the compounds of the formulae I', I'' and I''' are preferably pharmaceutically acceptable addition salts with non-toxic inorganic or organic acids. As examples of suitable acids hydrochloric, hydrobromic, sulphuric, nitric, phosphoric acid etc., and as organic acids for example, acetic, propionic, stearic, oxalic, malonic, succinic, glutaric, adipic, maleic, fumaric, malic, tartaric, citric, ascorbic, benzoic, pamoic or sulphonic acid, such as mesyl or tosyl acid, may be mentioned.

Especially advantageous compounds are the bispilocarpates according to the formula I', wherein Y and Y' are hydrogen and then specifically O,O'-dihydrogen (1,4-, 1,3-, 1,2-xylylene)- and (1,3-propylene) bispilocarpate.

Preferred diacyl bispilocarpates of the formula I' are O,O'-dipropionyl-, O,O'-dibutyryl-, and O,O'-dicyclo-propylcarbonyl (1,4-xylylene) bispilocarpate.

Preferred are also O,O'-dipropionyl (ethylene)- and O,O'-dipropionyl (2-hydroxy-1,3-propylene) bispilocarpate, and O,O'-dicyclopropylcarbonyl (1,5-pentylene) bispilocarpate and -(1,6-hexylene) bispilocarpate.

Preferred compounds of the formula I'' are the (dibenzyl) bispilocarpates, especially O,O'-glutaryl (dibenzyl) bispilocarpate. Preferred is also O,O'-succinyl (diethyl) bispilocarpate.

The invention relates also to a process for the preparation of the compounds according to the formulae I', I'' and I'''.

According to the process of the invention

a) for the preparation of a compound of the formula I' pilocarpic acid of the formula

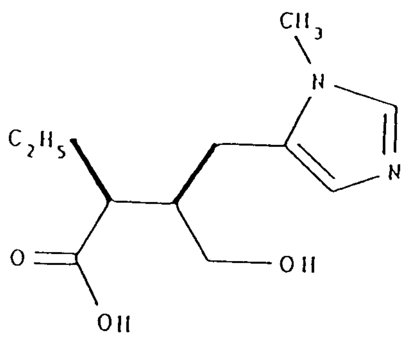

or its salt is reacted with a compound of the formula X-A-X', wherein X and X' are independently hydroxy, or a leaving group, such as halogen, acyloxy, or alkyl or aryl sulfonyloxy, for the preparation of a compound of the formula (II)

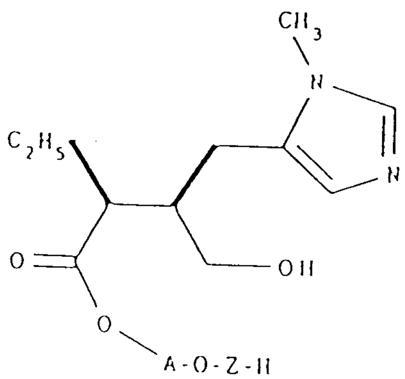

(II)

wherein Z is

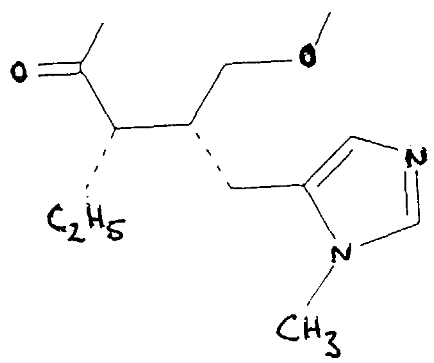

which, if desired, is converted to a compound of the formula I', wherein Y is different from hydrogen, by reacting the same with an acid of the formula $RCO_2H$, and when R is different from R', thereafter with an acid of the formula $R'CO_2H$, or with a functional derivative of these acids, and optionally the compound of the formula I' is converted to its acid addition salt, or

b) for the preparation of a compound of the formula I'', a compound of the formula III or III' respectively

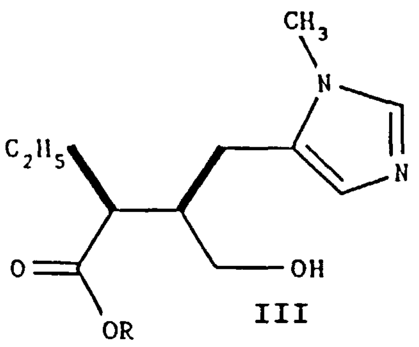

III

and/or

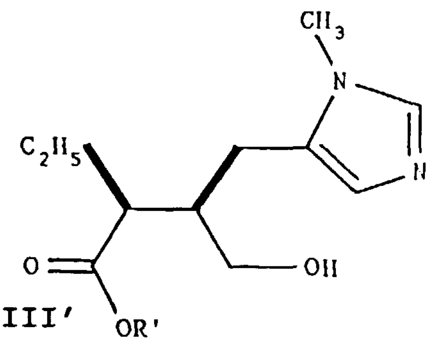

III'

wherein R and R' have the meaning given above, is reacted with a dicarboxylic acid of the formula IV

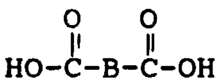

IV

wherein B has the meaning given above, or with a bifunctional acid derivative thereof, and optionally the compound obtained with the formula I'' is converted to its acid addition salt, or

c) for the preparation of a compound of the formula I''', a compound of the formula

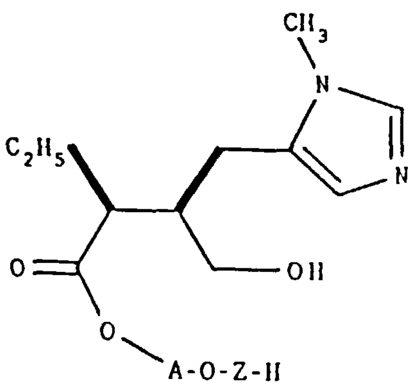

(II)

wherein A and Z have the meanings given above, is reacted with a dicarboxylic acid of the formula IV

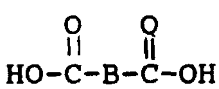

(IV)

wherein B has the meaning given above, or with a bifunctional acid derivative thereof, and optionally the obtained compound with the formula I''' is converted to its acid addition salt.

In the above mentioned reactions, as the functional acid derivatives preferably the halogenides, anhydrides, alkyl and aryl sulfonates thereof are used. As the reaction medium, solvents such as hydrocarbons, halogenated hydrocarbons, ethers, ketones etc are used, which are inert to the reagents. Suitable hydrocarbons are the aromatic hydrocarbons, such as benzene and alkyl benzenes, such as toluene and xylene. Suitable halogenated hydrocarbons are for

example dichloromethane, chloroform and chlorobenzene. As ketones acetone, ethylmethyl ketone and isobutylmethyl ketone may be mentioned, and as ethers diethyl ether, diisopropyl ether, dibutyl ether and 1,4-dioxane may be mentioned. Other suitable solvents include dimethyl sulfoxide, dimethylformamide and acetonitril.

The reaction temperature is not critical but may vary for example from -10°C to the boiling point of the solvent. Suitably room temperature is used. The reaction time may vary witih broad limits and is conventionally 12-72 hours, usually about 24 hours. It is suitable to use acid binding agents in the reactions, such as alkali metal and alkaline earth metal carbonates or organic bases. Suitable metal carbonates include sodium carbonate and potassium carbonate and as organic bases pyridine and its homologues, 4-(dimethyl amine) pyridine, quinoline and its homologues, N,N-dimethyl aniline and trialkyl amines, especially triethyl amine. The said reactions may take place either in a homogenous solution or in a heterogenic system, such as under PTC-conditions.

The ester bonds of the compounds according to the invention may be formed also by using known water cleaving reagents, such as carbodi-imides. In some cases also the known acid catalyzed esterification reactions may come into question.

The pharmaceutical compositions acccording to the invention are made in a known manner by using vehicles and other adjuvants known in the art. The vehicles may, for example, be liquids, suspensions or emulsions, or creams and ointments. The composition may also be formed into a solid pharmaceutical form to be inserted in the eye. A suitable form of administration is for example an eyedrop solution which contains the compound according to the invention at a suitable concentration, for example 0.1 to 4%, in a sterile aqueous solution buffered to a suitable pH or adjusted to a suitable pH with an acid or a base, the compound preferably being used in its water soluble acid addition salt form. An eyedrop solution with the said desired concentration is administered into the eye, depending on the patient, preferably 1 to 3 times a day.

TEST REPORT

TEST METHODS

The compounds of the invention have been tested in the following tests. Pilocarpine diesters known from the EP patent application EP-0 106 541 have been tested in the corresponding tests under the same conditions and compared to the bispilocarpates according to the invention.

1) Stability of the compounds

The stability of the test compounds were tested with buffer hydrolyses. The ionic strength (μ) of the buffers was 0.5, the pH-values used 4.2, 6.0, 7.4 and 9.0 and the temperatures used 37°C, 50 °C, 60 °C and 70 °C.

The half-times $T_{1/2}$ calculated on basis of the degradation constant k obtained for each studied compound ($T_{1/2}$=0.693/k; k = 2.303 x kk, wherein kk is the angular coefficient of the plot which illustrates the logarithm of the remaining ester as a function of time) in a buffer solution of said pH, are indicated in the Table I below, which values refer to half-times at 37°C.

The results in the Table I indicate that the bispilocarpates according to the invention are more stable in an acidic solution than in a basic, and at least as stable as the diesters according to the EP-patent application 0 106 541.

The storage stability of the derivatives under different storage conditions may be estimated by determining the degradation constants at different temperatures and calculating the degradation constant k at the desired temperature from the equation of the plot corresponding to the Arrhenius equation (1), wherein log k is given as a function of [1/T].

$$\text{Log } k = \text{Log } A - \frac{E_a}{2.303R} \times \frac{1}{T} \qquad (1)$$

From the degradation constant (k) obtained at the desired temperature the shelf-life-time $t_{10\%}$ ($t_{10\%}$ = 0.104/k) which indicates the time during which 10% of the drug has degraded may be calculated. By way of example it may be mentioned that the $t_{10}$%-time of O,O'-dipropionyl (1,4-xylylene) bispilocarpate at 4 °C is almost 500 days.

2) Lipophilicity

The lipophilicity of the compounds were studied by determining the partition coefficients (P) of the compounds at a pH of 7.40. The measurements were made either in an octanol-buffer mixture by determining the concentration of the compound to be studied in the buffer phase by HPLC. The partition coefficients of the very lipophilic

compounds were, however, determined by reverse phase (RP) liquid chromatography (HPLC) from the retention time (Beckmann 116 pump and 166 UV detector; Marathon autom. sample feeder).

The log P values and capacity factors k' of the compounds studied, pilocarpine and the bispilocarpates according to the invention, at 22 °C are given in the Table I below. From the results it can be seen that the compounds of the invention are considerably more lipophilic than the pilocarpine.

3) Enzyme hydrolysis

The half-times of enzyme hydrolysis of the novel bispilocarpates according to the invention and of known pilocarpic acid diesters were determined in a plasma/buffer pH 7.4-mixture (80%-20%) at 37°C. The degradation constants (k) for the compounds tested and determined from the plot showing the logarithm of remaining diester as a function of time and the half-lives $T_{1/2}$ are shown in the Table II below.

From the results it may be seen that in buffer solutions the stable test compound degrades under the influence of an enzyme (esterase) to a O,O'-dihydrogen bispilocarpate intermediate, which spontaneously degrades at pH 7.40 to pilocarpine. Consequently the effect of the bispilocarpate intermediate on the rate of formation of pilocarpine is very significant. The formation of pilocarpine was almost complete irrespective of the epimerization possibility of the intermediate. The amount of isopilocarpine formed varied from 0 to 10%, as also the pilocarpine formed epimerized, if the duration of hydrolysis was very long.

4) Corneal permeability

The corneal permeability of pilocarpine hydrochloride and its most promising prodrug derivatives as evaluated from the tests described above were studied in a diffusion chamber, wherein the migration of the compound from the delivering phase (epithelium side) thorugh the cornea to the acceptor side (endothelium side) of the diffusion chamber. In the study, rabbit eye cornea was used.

From the samples taken from the acceptor side of the diffusion chamber, both the concentration of the prodrug and the liberated drug were determined in the acceptor phase with HPLC. Thus the rate of degradation of the prodrug in the cornea was determined.

The permeability coefficients (Papp, cm/s) calculated from the permeability rate (µmol/min), their avarage deviations (RSD) and the number of tests, are given in the Table II below.

From the results can be seen that with the compounds from both groups it is possible to substantially improve the permeability of pilocarpine. The results also show that the pilocarpine esters degrade in the cornea forming pilocarpine and that with the groups attached to pilocarpic acid it is possible to regulate the corneal permeability of the prodrug and the rate of formation of pilocarpine.

**TABLE I**

| Compound | Half-times in buffers at 37°C | | | | Partition coefficient | Capacity factor (22°C) |
|---|---|---|---|---|---|---|
| | pH 4.20 | 6.00 | 7.40 | 9.00 | log P | k' |
| Pilocarpine | | | | | 0.0117[1] | 0.4051 |
| Compounds from EP 106541 | | | | | | |
| 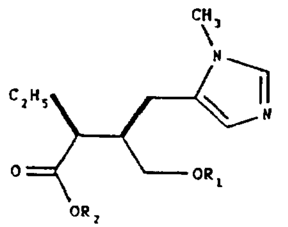 | | | | | | |
| $R_1$  $R_2$ | | $T_{½}$ (h) | | | | |
| acetyl benzyl | 7668 | 3466 | 547 | 79 | | |
| propionyl -"- | 2179 | 5335 | 748 | 94 | | |
| valeryl -"- | 8537 | - | 1169 | 303 | | |
| benzoyl -"- | 10667 | 4871 | 2043 | 111 | | |
| acetyl methyl-cyclohexyl | 2195 | 1663 | 503 | 73 | | |
| propionyl -"- | - | 1507 | 829 | 154 | | |

| Compounds according to the invention: | | | $T_{1/2}$ (min) | | | |
|---|---|---|---|---|---|---|
| O,O'-dihydrogen (1,4-xylylene) bispilocarpate | 2256 | 137 | 18 | 1.9 | 2.1753[1] | 0.8101 |
| O,O'-dihydrogen (1,3-xylylene) bispilocarpate | 2930 | 167 | 19 | 1.1 | 1.9901[1] | 0.8291 |
| O,O'-dihydrogen (1,2-xylylene) bispilocarpate | 1210 | 201 | 25 | 1.4 | 2.0252[1] | 0.8291 |
| O,O'-dihydrogen (1,7-heptylene) bispilocarpate | 4909 | 1792 | 201 | 18 | 2.6743[1] | 1.1266 |
| O,O'-dihydrogen (1,6-hexylene) bispilocarpate | 3759 | 1509 | 150 | 14 | 1.8360[1] | 0.8924 |
| O,O'-dihydrogen (1,5-pentylene) bispilocarpate | 4378 | 1474 | 125 | 14 | 1.5714[1] | 0.7532 |
| O,O'-dihydrogen (1,4-butylene) bispilocarpate | 7206 | 1136 | 158 | 10 | 1.1627[1] | 0.6582 |
| O,O'-dihydrogen (1,3-propylene) bispilocarpate | 4040 | 618 | 72 | 4 | 0.6830[1] | 0.6139 |
| | | | $T_{1/2}$ (h) | | | |
| O,O'-diacetyl (1,4-xylylene) bispilocarpate | 3964 | 1161 | 274 | 34 | 3.0432[1] | 1.3987 |
| O,O'-dipropionyl (1,4-xylylene) bispilocarpate | 2203 | 1214 | 400 | 47 | 4.0843[1] | 2.3038 |

| | | | | | | |
|---|---|---|---|---|---|---|
| O,O'-dibutyryl (1,4-xylylene) bispilocarpate | 5993 | 1114 | 177 | 137 | 5.5604[2] | 4.0127 |
| O,O'-divaleryl (1,4-xylylene) bispilocarpate | | | | 7 | 7.0165[2] | 7.3861 |
| O,O'-dicyclopropylcarbonyl (1,4-xylylene)-bispilocarpate | 63323 | 8445 | 3545 | 339 | 4.2015[1] | 2.4684 |
| O,O'-dibenzoyl (1,4-xylylene) bispilocarpate | | | | 8 | 6.4929[2] | 5.9304 |
| O,O'-succinyl (dibenzyl) bispilocarpate | 4761 | 2546 | 251 | 28 | 5.2038[2] | 3.4557 |
| O,O'-glutaryl (dibenzyl) bispilocarpate | 4187 | 2198 | 757 | 43 | 5.4093[2] | 3.7658 |
| O,O'-adipoyl (dibenzyl) bispilocarpate | 3141 | 1647 | 655 | 70 | 5.8242[2] | 4.4810 |
| O,O'-fumaroyl (dibenzyl) bispilocarpate | 834 | 49 | 6 | 0.4 | 5.7137[2] | 4.2785 |
| O,O'-terephtaloyl (dibenzyl) bispilocarpate | 1581 | 913 | 55 | 38 | 7.0000[2] | 7.3354 |

[1] partition coefficient determined with an octanol-buffer-mixture

[2] Log P determined from the equation log k'= 0.182 Log P - 0.4086

**TABLE II**

| Compound R1 | R2 | Degradation coefficient (k) and half-times ($T_{½}$) in plasma (80%), 37°C: k ($min^{-1}$) | $T_{½}$ (min) | Permeability coefficient: Papp ($cm/s \times 10^{-5}$) | Relative standard deviation (RSD) | Number of tests n |
|---|---|---|---|---|---|---|
| Pilocarpine | | | | 0.41 | (22.9) | 6 |
| Compounds from EP-106541 | | | | | | |
| acetyl | benzyl | 0.04353 | 16 | 1.17 | (14.0) | 6 |
| propionyl | -"- | 0.10801 | 6 | 1.43 | (26.8) | 6 |
| valeryl | -"- | 0.07876 | 9 | 1.15 | (26.4) | 6 |
| octanoyl | -"- | 0.02556 | 27 | | | |
| benzoyl | -"- | 0.03800 | 18 | 0.87 | (23.8) | 5 |
| 3-chloro-benzoyl | -"- | 0.03132 | 22 | 0.72 | (24.4) | 5 |
| acetyl | methyl-cyclohexyl | 0.00967 | 72 | | | |
| propionyl | -"- | 0.02957 | 33 | 0.13 | (29.3) | 6 |
| valeryl | -"- | 0.02487 | 28 | | | |
| benzoyl | -"- | 0.00599 | 116 | | | |
| 3-chloro-benzoyl | -"- | 0.00299 | 231 | | | |

| Compounds according to the invention | | | | | |
|---|---|---|---|---|---|
| O,O'-diacetyl (1,4-xylylene) bispilocarpate | 0.07370 | 9 | 0.93 | (33.2) | 6 |
| O,O'-dipropionyl (1,4-xylylene) bispilocarpate | 0.11837 | 6 | 3.05 | (14.7) | 6 |
| O,O'-dibutyryl (1,4-xylylene) bispilocarpate | 0.06149 | 11 | 2.15 | (34.0) | 9 |
| O,O'-divaleryl (1,4-xylylene) bispilocarpate | 0.05734 | 12 | 1.82 | (34.0) | 4 |
| O,O'-dicyclopropylcarbonyl (1,4-xylylene) bispilocarp | 0.04998 | 14 | 1.58 | (34.1) | 4 |
| O,O'-dibenzoyl (1,4-xylylene) bispilocarpate | 0.00737 | 94 | | | |
| O,O'-succinyl (dibenzyl) ~~(1,4-xyly-~~ bispilocarpate | 0.00368 | 188 | | | |
| O,O'-glutaryl (dibenzyl) bispilocarpate | 0.05044 | 14 | | | |
| O,O'-adipoyl (dibenzyl) bispilocarpate | 0.02487 | 28 | | | |
| O,O'-fumaroyl (dibenzyl) bispilocarpate | 0.00415 | 167 | | | |
| O,O'-terephtaloyl (dibenzyl) bispilocarpate | 0.00322 | 215 | | | |

5. Conclusion

The compounds of the invention have proven to be stable compounds also in water based solutions and they thus enable the preparation of eyedrop formulations. It has also been shown that by changing the groups attached to the pilocarpic acid, the lipophilicity, enzymatic degradation, corneal permeability of the derivative and thus the formation

of pilocarpine in the eye can be regulated easily according to purpose. The esterase enzymes of the eye initiate the hydrolysis of the bispilocarpates to an intermediate, which spontaneously degrades to pilocarpine.

When comparing the compounds according to the invention to the compounds according to the EP application 0 106 541, it is found that the compounds according to the invention are at least as good as the known compounds in all fields. A substantial advantage of the compounds according to the invention is, however, that the bispilocarpates of the invention produce two pilocarpine molecules for one bispilocarpate molecule, from which follows in turn relatively less products of metabolism split off from the prodrug-structure, especially monocarboxylic acids, aldehydes, etc. per pilocarpine molecule. By minimizing the amount of products of metabolism it is possible to reduce eye drug related irritation and stinging of the eye.

By means of the compounds of the invention it is possble to eliminate to a large extent the disadvantages relating to drug treatment with pilocarpine (poor biological availability, system and eye side-effects, frequent administration and thus poor patient compliance). Due to the better corneal permeability, the compounds of the invention may be administered in considerably smaller doses and the number of doses/day may be reduced, whereby the side-effects are reduced, patient compliance is improved and the drug treatment of the glaucoma patient is made more effective.

The following examples illustrate the invention without limiting the same in any way.

<u>EQUIPMENT USED</u>

Melting point determination:
Reichert Thermovar-apparatus
Determination of refraction index:
Atago Illuminator-apparatus
pKa-value determination:
titrating the derivative in a water-ethanol mixture (50%-50%)
Mass spectrometer:
VG 70-250SE
Test conditions in the electron bomb ionisator:

electron energy:
70 eV (unless otherwise mentioned)
ionisation current:
500 µA
ionisation chamber temperature:
150 °C
sample holder temperature
30 °C => 500 °C in 2-5 minutes
resolution:
10 000
Thermospray-mass spectrometer:
VG thermospray/plasmaspray
VG Trio-2 quadropoli
Beckmann 112 pump

Test conditions in the thermospray-ionisation optimized daily

NMR-spectrometer: Bruker AC 250/Aspect 3000
$^1H/^{13}C$ 5 mm dual probe
$CD_3OD$ 20 mg/ml
δ ppm (tetramethyl silane=0)

Example 1

O,O'-Dihydrogen (1,4-xylylene) bispilocarpate

(Formula I': Y=Y'=H; A=1,4-xylylene)

a) Pilocarpic acid sodium salt

Pilocarpine hydrochloride (3.92 g; 16.00 moles) was dissolved in distilled water (4 ml) and the solution was cooled to about 0 °C. To the solution 18 ml of ice cold 2M NaOH were added in four portions. The solution was left standing at about 0 °C for one hour. After neutralizing excess NaOH with 5 ml of 1M HCl, the solution was evaporated under reduced pressure. The residue was dissolved in 60 ml of absolute ethanol and mixed for 10 minutes at 60 °C. After cooling to 4 °C undissolved NaCl was removed by filtration. The filtrate was evaporated under reduced pressure, whereby 3.93 g of sodium pilocarpate were obtained as a white, extremely hygroscopic substance.

b) O,O'-Dihydrogen (1,4-xylylene) bispilocarpate

To a solution containing 8.00 mmoles (1987 mg) of pilocarpic acid sodium salt in 60 ml of dimethyl sulfoxide, 3.00 mmoles of α,α'-dichloro-p-xylene (524 mg) in dimethyl sulfoxide were added dropwise during about one hour. The solution was mixed at room temperature for 48-72 hours and poured into 100 ml of distilled water. The mixture was extracted with two portions of each 100 ml of chloroform. The combined chloroform extracts were washed with 100 ml of distilled water, with 100 ml of 2 % sodium bicarbonate solution and with 100 ml of distilled water. The chloroform extracts were dried on magnesium sulfate and the chloroform evaporated under reduced pressure, and the bispilocarpate obtained was crystallized from a chloroform/petrolether mixture, whereby 770 mg (1.39 mmoles) of the title compound were obtained.

M.p. 170-171 °C
$pK_a$ = 6.25
k' = 0.8101
HR-MS-spectrum: m/e (relative intensity): no $[M^{+\cdot}]$-peak, 208 (19%), 207 (6%), 96 (42%), 95 (100%).
HPLC-MS (thermospray): m/e (relative intensity): 555 [M+1] (12%), 417 (3%), 384 (4%), 347 (46%), 267 (10%), 250 (5%), 209 (100%).
NMR: δ 7.47 bs 2H, 7.39 s 4H, 6.69 bs 2H, 5.09 s 4H, 3.51 m 4H, 3.48 s 6H, 2.65 m 2H, 2.53 m 2H, 2.42 m 2H, 1.97 m 2H, 1.67 m 4H and 0.84 t 6H.

Example 2

O,O'-Dihydrogen (1,3-xylylene) bispilocarpate

(Formula I': Y=Y'=H; A=1,3-xylylene)

The compound was prepared from the sodium salt of pilocarpine (1287 mg; 5.18 mmoles) and α,α'-dibromo-m-xylene (342 mg; 1.30 mmoles) with the method disclosed in the Example 1. The bispilocarpate obtained was crystallized from a ethyl acetate/ether mixture. The yield was 380 mg (0.69 mmoles).

M.p. 116-117 °C
$pK_a$ = 6.30
k' = 0.8291
HR-MS-spectrum: m/e (relative intensity): no $[M^{+\cdot}]$-peak, 209 (11%), 208 (38%), 96 (44%), 95 (100%).
HPLC-MS (thermospray): m/e (relative intensity): 555 [M+1] (7%), 417 (7%), 385 (8%), 347 (46%), 267 (9%), 250 (10%), 209 (100%).
NMR: δ 7.47 bs 2H, 7.44 bs 1H, 7.35 m 3H, 6.70 bs 2H, 5.09 bs 4H, 3.52 m 4H, 3.48 s 6H, 2.66 m 2H, 2.52 m 2H, 2.43 m 2H, 1.97 m 2H, 1.67 m 4H and 0.85 t 6H.

Example 3

O,O'-Dihydrogen (1,2-xylylene) bispilocarpate

(Formula I': Y=Y'=H; A=1,2-xylylene)

The compound was prepared from the sodium salt of pilocarpine (1166 mg; 4.70 mmoles) and α,α'-dibromo-o-xylene (310 mg; 1.18 mmoles) with the method disclosed in the Example 1. The bispilocarpate obtained was crystallized from a ethyl acetate/petrolether mixture. The yield was 380 mg (0.69 mmoles).

M.p. 62-64 °C
$pK_a$ = 6.30
k' = 0.8291
HR-MS-spectrum: m/e (relative intensity): no $[M^{+\cdot}]$-peak, 208 (12%), 96 (19%), 95 (100%).
HPLC-MS (thermospray): m/e (relative intensity): 555 [M+1] (11%), 417 (7%), 385 (8%), 347 (46%), 267 (9%), 250 (14%), 209 (100%).
NMR: δ 7.47 bs 2H, 7.34 m 4H, 6.70 bs 2H, 5.24 s 4H, 3.52 m 4H, 3.48 s 6H, 2.66 m 2H, 2.53 m 2H, 2.42 m 2H, 1.97 m 2H, 1.67 m 4H and 0.85 t 6H.

Example 4

O,O'-Dihydrogen (1,7-heptylene) bispilocarpate

(Formula I': Y=Y'=H; A=1,7-heptylene)
The compound was prepared from the sodium salt of pilocarpine (1994 mg; 8.03 mmoles) and 1,7-dibromo heptane (518 mg; 2.01 mmoles) with the method disclosed in the Example 1. The bispilocarpate obtained was crystallized from a chloroform/petrolether mixture. The yield was 1050 mg (1.91 mmoles).

M.p. 117-120 °C
$pK_a$ = 6.30
k' = 1.1266
HR-MS-spectrum: m/e (relative intensity):
208 (16%), 96 (2%), 95 (100%).
HPLC-MS (thermospray): m/e (relative intensity): 549 [M+1] (4%), 341 (16%), 267 (7%), 250 (5%), 209 (100%).
NMR: δ 7.50 bs 2H, 6.73 bs 2H, 4.05 t 4H, 3.59 s 6H, 3.53 m 4H, 2.72 m 2H, 2.50 m 2H, 2.47 m 2H, 1.99 m 2H, 1.68 m 4H, 1.64 bm 8H, 1.37 m 2H and 0.88 t 6H.

Example 5

O,O'-Dihydrogen (1,6-hexylene) bispilocarpate

(Formula I': Y=Y'=H; A=1,6-hexylene)
The compound was prepared from the sodium salt of pilocarpine (1353 mg; 5.45 mmoles) and 1,6-dibromo hexane (499 mg; 2.04 mmoles) with the method disclosed in the Example 1. The bispilocarpate obtained was crystallized from a chloroform/petrolether mixture. The yield was 717 mg (1.34 mmoles).

M.p. 115-118 °C
$pK_a$ = 6.30
k' = 0.8924
HR-MS-spectrum: m/e (relative intensity): no $[M^{+\cdot}]$-peak, 208 (16%), 96 (24%), 95 (100%).
HPLC-MS (thermospray): m/e (relative intensity): 535 [M+1] (5%), 327 (26%), 267 (13%), 250 (7%), 209 (100%).
NMR: δ 7.49 bs 2H, 6.73 bs 2H, 4.05 t 4H, 3.60 s 6H, 3.53 m 4H, 2.72 m 2H, 2.51 m 2H, 2.47 m 2H, 1.99 m 2H, 1.67 m 4H, 1.65 m 4H, 1.41 m 4H and 0.88 t 6H.

Example 6

O,O'-Dihydrogen (1,5-pentylene) bispilocarpate

(Formula I': Y=Y'=H; A=1,5-pentylene)
The compound was prepared from the sodium salt of pilocarpine (1051 mg; 4.23 mmoles) and 1,5-dibromo pentane (244 mg; 1.06 mmoles) with the method disclosed in the Example 1. The bispilocarpate obtained was crystallized from a ethyl acetate/petrolether mixture. The yield was 395 mg (0.75 mmoles).

M.p. 84-87 °C

$pK_a = 6.30$
k' = 0.7532
HR-MS-spectrum: m/e (relative intensity): 209 (8%), 208 (9%), 96 (27%), 95 (100%).
HPLC-MS (thermospray): m/e (relative intensity): 521 [M+1] (15%), 417 (6%), 351 (9%), 313 (73%), 267 (11%), 250 (12%), 209 (100%).
NMR: δ 7.50 bs 2H, 6.73 bs 2H, 4.07 t 4H, 3.60 s 6H, 3.55 m 4H, 2.73 m 2H, 2.52 m 2H, 2.48 m 2H, 1.99 m 2H, 1.68 m 4H, 1.64 m 4H, 1.52 m 2H and 0.89 t 6H.

Example 7

O,O'-Dihydrogen (1,4-butylene) bispilocarpate

(Formula I': Y=Y'=H; A=1,4-butylene)

The compound was prepared from the sodium salt of pilocarpine (1069 mg; 4.31 mmoles) and 1,4-dibromo butane (233 mg; 1.08 mmoles) with the method disclosed in the Example 1. The bispilocarpate obtained was crystallized from a chloroform/ether/ethyl acetate mixture. The yield was 185 mg (0.37 mmoles).

M.p. 127-129 °C
$pK_a = 6.35$
k' = 0.6582
HR-MS-spectrum: m/e (relative intensity): 299 (21%), 267 (11%), 209 (20%), 208 (7%), 139 (49%), 96 (39%), 95 (100%).
HPLC-MS (thermospray): m/e (relative intensity): 507 [M+1] (18%), 417 (6%), 337 (6%), 299 (66%), 267 (11%), 250 (11%), 209 (100%).
NMR: δ 7.50 bs 2H, 6.73 bs 2H, 4.08 t 4H, 3.60 s 6H, 3.54 m 4H, 2.73 m 2H, 2.52 m 2H, 2.47 m 2H, 2.00 m 2H, 1.81 m 4H, 1.68 m 4H and 0.89 t 6H.

Example 8

O,O' -Dihydrogen (1,3-propylene) bispilocarpate

(Formula I': Y=Y'=H; A=1,3-propylene)

The compound was prepared from the sodium salt of pilocarpine (1021 mg; 4.11 mmoles) and 1,3-dibromo propane (208 mg; 1.03 mmoles) with the method disclosed in the Example 1. The bispilocarpate obtained was crystallized from an ethyl acetate/ether mixture. The yield was 330 mg (0.70 mmoles).

M.p. 71-73 °C
$pK_a = 6.40$
k' = 0.6139
HR-MS-spectrum: m/e (relative intensity): 303 (4%), 209 (46%), 208 (15%), 96 (27%), 95 (100%).
HPLC-MS (thermospray): m/e (relative intensity): 493 [M+1] (25%), 417 (7%), 323 (9%), 285 (73%), 267 (9%), 250 (10%), 209 (100%).
NMR: δ 7.50 bs 2H, 6.72 bs 2H, 4.19 t 4H, 3.60 s 6H, 3.54 m 4H, 2.72 m 2H, 2.53 m 2H, 2.49 m 2H, 1.99 m 2H, 2.08 qv 2H, 1.68 m 4H and 0.89 t 6H.

Example 9

O,O'-Diacetyl (1,4-xylylene) bispilocarpate

(Formula I': Y=Y'=acetyl, A=1,4-xylylene; fumarate)

Into a mixture containing 679 mg (1.22 mmoles) of O,O'-dihydrogen (1,4-xylylene) bispilocarpate (see Example 1) and 2026 mg of potassium carbonate (14.66 mmoles) in toulene (150 ml), 767 mg (9.77 mmoles) of acetyl chloride were added dropwise during 30 to 40 hours. The mixture was stirred for 40 to 72 hours. To the reaction mixture a 2 % sodium bicarbonate solution (150 ml) was added and the mixture was stirred at room temperature for 3 hours. The layers were separated and the toluene phase washed twice with water (2 x 150 ml), was dried on calcium sulfate (30 min) and evaporated under reduced pressure, whereby O,O'-diacetyl (1,4-xylylene) bispilocarpate (free base) was obtained as an oil (780 mg, 1.22 mmoles). The oil was dissolved in toluene (20 ml) and a solution of fumaric acid (425 mg, 3.66 mmoles) in 2-propanol (10 ml) was added. The salt was precipitated with petrol ether. The mixture was

allowed to stand over night whereafter the O,O'-diacetyl (1,4-xylylene) bispilocarpate fumarate was isolated by filtering, whereby 750 mg (0.76 mmoles) of the fumarate salt were obtained.

$n_d^{20}$ = 1.5230 (free base)
M.p. 58-60 °C
$pK_a$ = 6.03
k' = 1.3987
HR-MS-spectrum: m/e (relative intensity): 638 ($M^{+\cdot}$) (5%), 565 (3%), 458 (22%), 443 (19%), 387 (24%), 208 (21%), 207 (45%), 96 (20%), 95 (100%).

HR-MS = molecular weight =
638.32739 (measured)
638.33156 (calculated)

HPLC-MS (thermospray): m/e (relative intensity): 677 (35%), 661 (21%), 639 [M+1] (100%), 469 (13%), 431 (50%), 389 (37%), 209 (55%).
NMR: δ 8.46 bs 2H, 7.41 s 4H, 7.19 bs 2H, 6.71 (fum.), 5.15 m 4H, 4.06 m 4H, 3.71 s 6H, 2.71 m 4H, 2.56 m 2H, 2.36 m 2H, 2.00 s 6H, 1.69 m 4H and 0.86 t 6H.

Example 10

O,O'-Dipropionyl (1,4-xylylene) bispilocarpate

(Formula I': Y=Y'=propionyl; A=1,4-xylylene; fumarate)
The compound was prepared from O,O'-dihydrogen (1,4-xylylene) bispilocarpate (473 mg; 0.85 mmoles) (see Example 1) and propionyl chloride (631 mg; 6.82 mmoles) according to the method described in the Example 9. The compound was crystallized from a 2-propanol/toluene/petrolether mixture. The yield was 730 mg (0.72 mmoles).

$n_d^{20}$ = 1.5205 (free base)
M.p. 86-89 °C
$pK_a$ = 5.80
k' = 2.3038
HR-MS-spectrum: m/e (relative intensity): 666 ($M^{+\cdot}$)(5%), 472 (53%), 457 (45%), 401 (58%), 209 (14%), 208 (13%), 207 (34%), 96 (43%), 95 (100%).

HR-MS = molecular weight =
666.35667 (measured)
666.36296 (calculated)

HPLC-MS (thermospray): m/e (relative intensity): 705 (46%), 689 (22%), 667 [M+1] (100%), 459 (18%), 404 (11%), 351 (41%), 209 (80%).
NMR: δ 8.39 bs 2H, 7.41 s 4H, 7.15 bs 2H, 6.72 (fum.), 5.15 m 4H, 4.07 m 4H, 3.69 s 6H, 2.71 m 4H, 2.55 m 2H, 2.36 m 2H, 2.30 q 4H, 1.69 m 4H, 1.08 t 6H, 0.86 t 6H.

Example 11

O,O'-Dibutyryl (1,4-xylylene) bispilocarpate

(Formula I': Y=Y'=butyryl; A=1,4-xylylene; fumarate)
The compound was prepared from O,O'-dihydrogen (1,4-xylylene) bispilocarpate (431 mg; 0.78 mmoles) (see Example 1) and butyryl chloride (663 mg; 6.22 mmoles) according to the method described in the Example 9. The compound was crystallized from a 2-propanol/toluene/petrolether mixture. The yield was 800 mg (0.77 mmoles).

$n_d^{20}$ = 1.5070 (free base)
M.p. = 90-92 °C
$pK_a$ = 6.00
k' = 4.0127
HR-MS-spectrum: m/e (relative intensity): 694 ($M^{+\cdot}$)(3%), 486 (26%), 471 (14%), 415 (17%), 209 (15%), 208

(20%), 207 (37%), 96 (26%), 95 (100%).

HR-MS = molecular weight = 694.39378 (measured)
694.39415 (calculated)

HPLC-MS (thermospray): m/e (relative intensity): 732 (77%), 717 (36%), 695 [M+1] (100%), 525 (9%), 487 (13%), 455 (7%), 417 (14%), 297 (7%), 209 (28%).
NMR: δ 8.51 bs 2H, 7.41 s 4H, 7.21 bs 2H, 6.71 (fum.), 5.15 m 4H, 4.07 m 4H, 3.72 s 6H, 2.72 m 4H, 2.55 m 2H, 2.36 m 2H, 2.27 t 4H, 1.69 m 4H, 1.59 m 4H, 0.91 t 6H and 0.86 t 6H.

Example 12

O,O'-Divaleryl (1,4-xylylene) bispilocarpate

(Formula I': Y=Y'=valeryl; A=1,4-xylylene; fumarate)
The compound was prepared from O,O'-dihydrogen (1,4-xylylene) bispilocarpate (395 mg; 0.71 mmoles) (see Example 1) and valeryl chloride (685 mg; 5.68 mmoles) according to the method described in the Example 9. The compound was recrystallized from a 2-propanol/toluene/petrolether mixture. The yield was 567 mg (0.53 mmoles).

$n_d^{20}$ = 1.5080 (free base)
M.p. = 84-86 °C
$pK_a$ = 6.00
k' = 7.3861
HR-MS-spectrum: m/e (relative intensity): 722 ($M^{+\cdot}$) (9%), 500 (60%), 485 (31%), 429 (41%), 209 (14%), 208 (17%), 207 (34%), 96 (46%), 95 (100%).

HR-MS = molecular weight =
722.42308 (measured)
722.42546 (calculated)

HPLC-MS (thermospray): m/e (relative intensity): 761 (86%), 745 (33%), 723 [M+1] (100%), 431 (16%), 362 (9%), 209 (35%).
NMR: δ 8.35 bs 2H, 7.41 s 4H, 7.13 bs 2H, 6.72 (fum.), 5.15 m 4H, 4.07 m 4H, 3.69 s 6H, 2.70 m 4H, 2.55 m 2H, 2.36 m 2H, 2.29 t 4H, 1.69 m 4H, 1.55 m 4H, 1.33 m 4H, 0.91 t 6H and 0.86 t 6H.

Example 13

O,O'-Dicyclopronylcarbonyl (1,4-xylylene) bispilocarpate

(Formula I': Y=Y'=cyclopropylcarbonyl, A=1,4-xylylene; fumarate)
The compound was prepared from O,O'-dihydrogen (1,4-xylylene) bispilocarpate (547 mg; 0.99 mmoles) (see Example 1) and cyclopropylcarbonyl chloride (788 mg; 7.92 mmoles) according to the method described in the Example 9. The compound was crystallized from a 2-propanol/toluene/petrol-ether mixture. The yield was 843 mg (0.81 mmoles).

$n_d^{20}$ = 1.5290 (free base)
M.p. = 73-75 °C
$pK_a$ = 6.00
k' = 2.4684
HR-MS-spectrum: m/e (relative intensity): 690 ($M^{+\cdot}$) (5%), 484 (36%), 469 (19%), 413 (30%), 209 (7%), 208 (16%), 207 (46%), 96 (30%), 95 (100%).

HR-MS = molecular weight =
690.36014 (measured)
690.36286 (calculated)

HPLC-MS (thermospray): m/e (relative intensity): 728 (89%), 713 (42%), 691 [M+1] (100%), 623 (12%), 521 (23%), 480 (30%), 453 (32%), 415 (46%), 209 (73%).

NMR: δ 8.55 bs 2H, 7.41 s 4H, 7.24 bs 2H, 6.71 (fum.), 5.16 m 4H, 4.08 m 4H, 3.73 s 6H, 2.74 m 4H, 2.57 m 2H, 2.38 m 2H, 1.70 m 4H, 1.69 m 2H, 0.87 t 6H and 0.86 m 8H.

Example 14

O,O'-Dibenzoyl (1,4-xylylene) bispilocarpate

(Formula I': Y=Y'=benzoyl, A=1,4-xylylene; fumarate)

The compound was prepared from O,O'-dihydrogen (1,4-xylylene) bispilocarpate (486 mg; 0.88 mmoles) (see Example 1) and benzoyl chloride (990 mg; 7.04 mmoles) according to the method described in the Example 9. The compound was crystallized from a 2-propanol/toluene/petrolether mixture. The yield was 464 mg (0.42 mmoles).

$n_d^{20}$ = 1.5605 (free base)
M.p. = 72-75 °C
$pK_a$ = 5.80
k' = 5.9304
HR-MS-spectrum: m/e (relative intensity): 762 ($M^{+\cdot}$) (1%), 243 (10%), 208 (15%), 207 (38%), 96 (26%), 95 (100%).

HR-MS = molecular weight =
762.36774 (measured)
762.36286 (calculated)

HPLC-MS (thermospray): m/e (relative intensity): 785 (28%), 763 [M+1] (100%), 593 (16%), 555 (21%), 489 (7%), 451 (8%), 209 (42%).
NMR: δ 8.41 bs 2H, 7.93 m 4H, 7.60 m 2H, 7.46 m 4H, 7.32 s 4H, 7.20 bs 2H, 6.71 (fum.), 5.07 m 4H, 4,32 m 4H, 3.71 s 6H, 2.82 m 4H, 2.65 m 2H, 2.53 m 2H, 1.75 m 4H and 0.89 t 6H.

Example 15

O,O'-Adipoyl (dibenzyl) bispilocarpate

(Formula I": R=R'=benzyl, B=1,4-butylene; fumarate)

Into a solution containing 7.78 mmoles (1932 mg) of pilocarpic acid sodium salt, the preparation of which is disclosed in the Example 1, in 60 ml of dimethyl sulfoxide, 7.78 mmoles of benzyl chloride (985 mg) were added within about one hour. The solution was stirred at room temperature for 48 to 72 hours, and poured into 100 ml of distilled water. The mixture was extracted with two portions of 100 ml ethyl acetate. The combined ethyl acetate extracts were washed with 150 ml of distilled water, 150 ml of a 2 % sodium bicarbonate solution and 150 ml of distilled water. The ethyl acetate extracts were dried with calcium sulfate and the ethyl acetate was evaporated under reduced pressure to produce pilocarpic acid benzyl ester. The ester was crystallized from a chloroform/petrolether mixture, whereby 1125 mg (5.56 mmoles) of the ester were obtained.

M.p. = 101-104 °C
$pK_a$ = 6.50
k' = 0.7658
HR-MS-spectrum: m/e (relative intensity): 208 (15%), 96 (45%), 95 (100%).
HR-MS = molecular weight = no $M^{+\cdot}$-peak
HPLC-MS (thermospray): m/e (relative intensity): 317 [M+1] (51%), 209 (100%)
NMR: δ 7.47 bs 1H, 7.33 m 5H, 6.69 bs 1H, 5.10 s 2H, 3.52 m 2H, 3.47 s 3H, 2.70 m 1H, 2.52 m 1H, 2.45 m 1H, 1.98 m 1H, 1.70 m 2H and 0.86 t 3H.

Into a mixture containing 406 mg (1.28 mmoles) of pilocarpic acid benzyl ester and 553 mg (3.00 mmoles) of potassium carbonate in toluene (40 ml) adipoyl chloride (108 mg; 0.59 mmoles) was added dropwise within about 24 hours. The solution was stirred at room temperature about for 48 hours. To the reaction mixture a 2 % sodium bicarbonate solution (40 ml) was added and the mixture was stirred at room temperature for 3 hours. The layers were separated and the toluene phase was washed twice with distilled water (2 x 50 ml), dried on calcium sulfate (30 min) and evaporated under reduced pressure, whereby O,O'-adipoyl (dibenzyl) bispilocarpate (free base) was obtained as an oil (324 mg; 0.44 mmoles). The oil was dissolved in toluene (15 ml) and a solution of fumaric acid (153 mg; 1.32 mmoles) in 2-propanol (5 ml) was added. The salt was precipitated with petrolether. The mixture was allowed to stand over night

whereby O,O'-adipoyl (dibenzyl) bispilocarpate fumarate (327 mg; 0.30 mmoles) was obtained.

$n_d^{20}$ = 1.5340 (free base)
M.p. = 98-100 °C
$pK_a$ = 6.00
k' = 4.4810
HR-MS-spectrum: m/e (relative intensity): 742 ($M^{+\cdot}$)(5%), 741 (8%), 629 (39%), 625 (52%), 536 (75%), 535 (100%), 459 (69), 445 (43%), 357 (33%), 299 (45%), 209 (55%), 207 (64%), 95 (69%), 91 (56%).

HR-MS = molecular weight =
742.390380 (measured)
742.394150 (calculated)

HPLC-MS (thermospray): m/e (relative intensity): 780 (75%), 765 (28%), 743 [M+1] (100%), 480 (8%), 445 (10%), 317 (23%), 209 (26%).
NMR: δ 8.36 bs 2H, 7.35 m 10H, 7.14 bs 2H, 6.72 (fum.), 5.14 m 4H, 4.07 m 4H, 3.65 s 6H, 2.68 m 4H, 2.54 m 2H, 2.34 m 2H, 2.30 t 4H, 1.69 m 4H, 1.59 t 4H and 0.87 t 6H.

Example 16

O,O'-Glutaryl (dibenzyl) bispilocarpate

(Formula I'': R=R'=benzyl, B=1,3-propylene; fumarate)

The compound was prepared from pilocarpic acid benzyl ester (633 mg; 2.00 mmoles) and glutaryl chloride (135 mg; 0.80 mmoles) according to the method described in the Example 15. The compound was crystallized from a 2-propanol/toluene/petrolether mixture. The yield was 477 mg (0.44 mmoles).

$n_d^{20}$ = 1.5330 (free base)
M.p. = 55-58 °C
$pK_a$ = 6.00
k' = 3.7658
HR-MS-spectrum: m/e (relative intensity): 728 ($M^{+\cdot}$)(0,15%), 419 (8%), 343 (16%), 209 (12%), 208 (14%), 207 (45%), 96 (31%), 95 (76%), 91 (100%)

HR-MS = molecular weight =
728.3734740 (measured)
728.3785152 (calculated)

HPLC-MS (thermospray): m/e (relative intensity): 729 [M+1] (36%), 431 (45%), 317 (35%), 209 (100%).
NMR: δ 8.47 bs 2H, 7.35 m 10H, 7.20 bs 2H, 6.71 (fum.), 5.14 m 4H, 4.07 m 4H, 3.67 s 6H, 2.68 m 4H, 2.54 m 2H, 2.34 t 4H, 2.32 m 2H, 1.84 m 2H, 1.69 m 4H and 0.87 t 6H.

Example 17

O,O'-Succinyl (dibenzyl) bispilocarpate

(Formula I'': R=R'=benzyl, B=ethylene; fumarate)

The compound was prepared from pilocarpic acid benzyl ester (633 mg; 2.00 mmoles) and succinyl chloride (124 mg; 0.80 mmoles) according to the method described in the Example 15. The compound was crystallized from a 2-propanol/toluene/petrolether mixture. The yield was 402 mg (0.38 mmoles).

$n_d^{20}$ = 1.5360 (free base)
M.p. = 65-67 °C
$pK_a$ = 6.00
k' = 3.4557
HR-MS-spectrum: m/e (relative intensity): 714 ($M^{+\cdot}$)(1%), 537 (10%), 415 (9%), 329 (11%), 208 (10%), 207 (31%), 96 (23%), 95 (94%), 91 (100%)

HR-MS = molecular weight =
714.3668980 (measured)
714.3628652 (calculated)

HPLC-MS (thermospray): m/e (relative intensity): 751 (10%), 715 [M+1] (47%), 417 (32%), 317 (57%), 209 (100%).
NMR: δ 8.44 bs 2H, 7.35 m 10H, 7.17 bs 2H, 6.71 (fum.), 5.14 m 4h, 4.09 m 4H, 3.65 s 6H, 2.68 m 4H, 2.58 s 4H, 2.53 m 2H, 2.32 m 2H, 1.69 m 4H and 0.86 t 6H.

Example 18

O,O'-Fumaroyl (dibenzyl) bispilocarpate

(Formula 1'': R=R'=benzyl, B=ethenylene; fumarate)

The compound was prepared from pilocarpic acid benzyl ester (633 mg; 2.00 mmoles) and fumaroyl chloride (122 mg; 0.80 mmoles) according to the method described in the Example 15. The compound was crystallized from a 2-propanol/toluene/petrolether mixture. The yield was 112 mg (0.11 mmoles).

$n_d^{20}$ = 1.5415 (free base)
M.p. = 64-66 °C
$pK_a$ = 6.05
k' = 4.2785
HR-MS-spectrum: m/e (relative intensity): 712 ($M^{+\cdot}$)(0,4%), 411 (41%), 407 (34%), 318 (56%), 317 (96%), 299 (39%), 209 (67%), 207 (40%), 96 (47%), 95 (100%), 91 (62%).

HR-MS = molecular weight =
712.3462220 (measured), 20 eV
712.3472151 (calculated)

HPLC-MS (thermospray): m/e (relative intensity): 750 (40%), 733 (20%), 713 [M+1] (100%), 450 (15%), 413 (32%), 317 (96%), 209 (42%).

Example 19

O,O'-Terephtaloyl (dibenzyl) bispilocarpate

(Formula I'': R=R'=benzyl, B=p-phenylene; fumarate)

The compound was prepared from pilocarpic acid benzyl ester (633 mg; 2.00 mmoles) and terephtaloyl chloride (162 mg; 0.80 mmoles) according to the method described in the Example 15. The compound was crystallized from a 2-propanol/toluene/petrolether mixture. The yield was 370 mg (0.33 mmoles).

$n_d^{20}$ = 1.5510 (free base)
M.p. = 130-133 °C
$pK_a$ = 6.00
k' = 7.3354
HR-MS-spectrum: m/e (relative intensity): 762 ($M^{+\cdot}$)(1%), 208 (13%), 207 (46%), 96 (36%), 95 (100%), 91 (70%).

HR-MS = molecular weight =
762.3563840 (measured)
762.3628652 (calculated)

HPLC-MS (thermospray): m/e (relative intensity): 763 [M+1] (82%), 465 (52%), 317 (97%), 267 (23%), 209 (100%).
NMR: δ 8.46 bs 2H, 8.05 s 4H, 7.32 m 10H, 7.24 bs 2H, 6.72 (fum.), 5.10 m 4H, 4.37 m 4H, 3.70 s 6H, 2.83 m 4H, 2.66 m 2H, 2.54 m 2H, 1.75 m 4H and 0.91 t 6H.

Example 20

O,O'-Dicyclopropylcarbonyl (1,6-hexylene) bispilocarpate

(Formula I': Y=Y'=cyclopropylcarbonyl, A=1,6-hexylene; fumarate)

The compound was prepared from O,O'-dihydrogen (1,6-hexylene) bispilocarpate (600 mg; 1.12 mmoles) (see Example 5) and cyclopropylcarbonyl chloride (766 mg; 7.33 mmoles) according to the method described in the Example 9. The compound was crystallized from a 2-propanol/toluene/petrol-ether mixture. The yield was 620 mg (0.61 mmoles).

$n_d^{20}$ = 1.5020 (free base)
M.p. = 60-63 °C (fumarate salt)
$pK_a$ = 6.00 (free base)
HR-MS-spectrum: m/e (relative intensity): 670 ($M^{+\cdot}$) (2%), 464 (13%), 449 (10%), 337 (10%), 209 (10%), 207 (21%), 96 (53%), 95 (100%).
HR-MS = molecular weight =
HPLC-MS (thermospray): m/e (relative intensity): 671 [M+1] (100%),
NMR: δ 8.53 bs 2H, 7.24 bs 2H, 6.72 (fum.), 4.12 bm 8H, 3.81 s 6H, 2.77 m 4H, 2.52 m 2H, 2.38 m 2H, 1.70 m 8H, 1.61 m 2H, 1.45 m 4H, 0.91 m 6H, 0.90 m 8H.
Enzyme hydrolysis in 80% plasma (pH 7.40 and 37 °C): $t_{1/2}$=15 min

| | |
|---|---|
| Chemical hydrolysis (50 °C): | pH 7.40: $t_{1/2}$=664 t |
| | pH 6.00: $t_{1/2}$=3405 t |
| | pH 4.20: $t_{1/2}$=3221 t |

Log(P)=0.880 (pH 5.0)

Example 21

O,O'-Dicyclopropylcarbonyl (1,5-pentylene) bispilocarpate

(Formula I': Y=Y'=cyclopropylcarbonyl, A=1,5-pentylene; fumarate)

The compound was prepared from O,O'-dihydrogen (1,5-pentylene) bispilocarpate (424 mg; 0.82 mmoles) (see Example 6) and cyclopropylcarbonyl chloride (682 mg; 6.52 mmoles) according to the method described in the Example 9. The compound was crystallized from a 2-propanol/toluene/petrolether mixture. The yield was 230 mg (0.23 mmoles).

$n_d^{20}$ =
M.p. = 56-59 °C
$pK_a$ =
HR-MS-spectrum: m/e (relative intensity): 656 ($M^{+\cdot}$) (3%), 450 (39%), 435 (23%), 379 (13%), 363 (13%), 209 (15%), 207 (29%), 96 (50%), 95 (100%).
HPLC-MS (thermospray): m/e (relative intensity): 657 [M+1] (92%),
NMR: δ 8.34 bs 2H, 7.15 bs 2H, 6.71 (fum.), 4.12 bm 8H, 3.77 s 6H, 2.77 m 4H, 2.51 m 2H, 2.38 m 2H, 1.71 m 8H, 1.61 m 2H, 1.48 m 4H, 0.91 m 6H, 0.88 m 8H.
Enzyme hydrolysis in 80% plasma (pH 7.40 and 37 °C): $t_{1/2}$=13 min

| | |
|---|---|
| Chemical hydrolysis (50 °C): | pH 7.40: $t_{1/2}$=553 t |
| | pH 6.00: $t_{1/2}$=2368 t |
| | pH 4.20: $t_{1/2}$=4624 t |

Log(P)=0.401 (pH 5.0)

**Claims**

**1.** Bispilocarpic acid ester derivatives of the formulae I', I'' and I'''

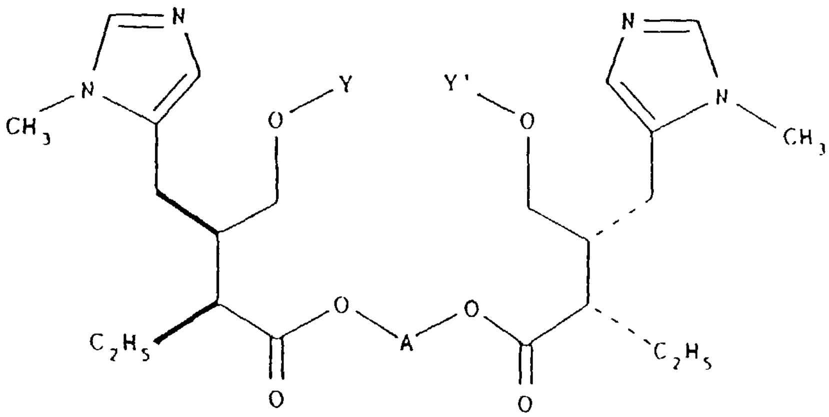

(I')

wherein

Y is hydrogen or

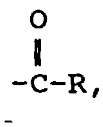

wherein R is hydrogen, $C_1$-$C_{18}$-alkyl, $C_2$-$C_{18}$-alkenyl, $C_2$-$C_{18}$-alkynyl, unsubstituted or $C_1$-$C_4$-alkyl substituted $C_3$-$C_7$-cycloalkyl or $C_3$-$C_7$-cycloalkenyl, unsubstituted or $C_1$-$C_4$-alkyl, $C_1$-$C_4$-alkoxy, nitro or halogen substituted aryl or aryl lower alkyl with up to 6 carbons in the alkyl moiety, and

Y' has the meaning of hydrogen or the group

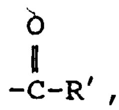

wherein R' has the meaning of R above, whereby R' can be the same as or different from R, A is optionally hydroxy or protected-hydroxy substituted $C_1$-$C_{18}$-alkylene, $C_2$-$C_{18}$-alkenylene or $C_2$-$C_{18}$-alkynylene, which may be substituted by unsubstituted or $C_1$-$C_4$-alkyl substituted $C_3$-$C_7$-cycloalkyl, $C_3$-$C_7$-cycloalkenyl, unsubstituted or $C_1$-$C_4$-alkyl, $C_1$-$C_4$-alkoxy, nitro or halogen substituted aryl or aryl lower alkyl, with up to 6 carbons in the alkyl moiety, or A is unsubstituted or $C_1$-$C_4$-alkyl substituted $C_3$-$C_7$-cycloalkylene or $C_3$-$C_7$-cycloalkenylene, or unsubstituted or $C_1$-$C_4$-alkyl, $C_1$-$C_4$-alkoxy, nitro or halogen substituted arylene, or A is the aforementioned alkylene, alkenylene, or alkynylene, which as a chain member contains the afore defined cycloalkylene, cycloalkenylene or arylene group,

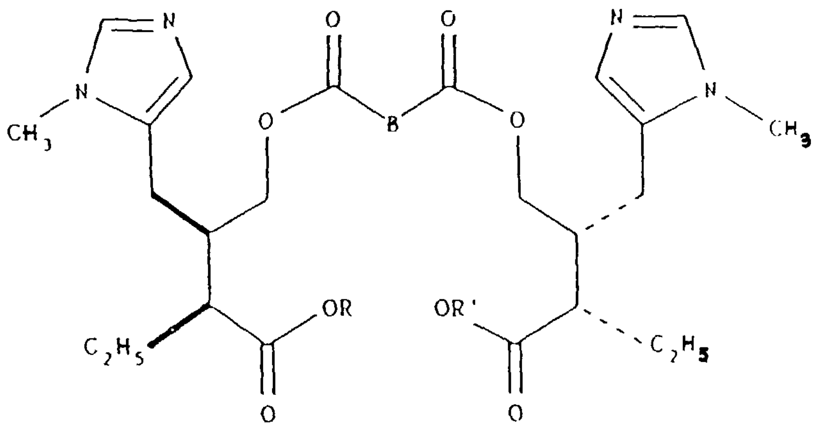

(I")

wherein R has the meaning given above,

R' has the meaning given above and B has the meaning given for A above, and

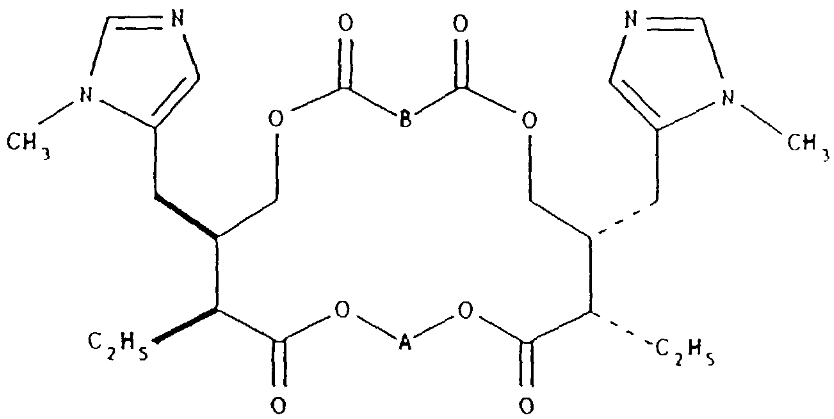

(I"')

wherein A and B have the meaning given above, as well as the acid addition salts of the said compounds.

2. Bispilocarpine derivative according to the Claim 1, **characterized** in that it is a (xylylene) bispilocarpate, especially (1,4-xylylene) bispilocarpate.

3. Bispilocarpine derivative according to the Claim 1, **characterized** in that it is O,O'-dipropionyl, O,O'-dibutyryl or O,O'-dicyclopropylcarbonyl (1,4-xylylene) bispilocarpate.

4. Bispilocarpine derivative according to the Claim 1, **characterized** in that it is a (dibenzyl) bispilocarpate, especially O,O'-glytaryl (dibenzyl) bispilocarpate.

5. Process for the preparation of the compounds according to the formulae I', I" and I"' in Claim 1, **characterized** in that

a) for the preparation of a compound of the formula I' pilocarpic acid of the formula

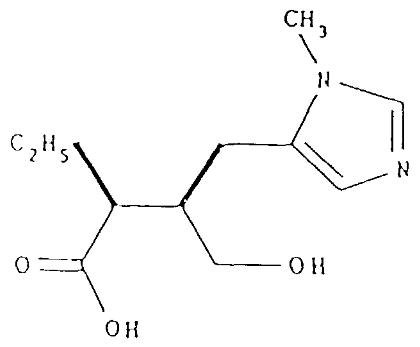

or its salt is reacted with a compound of the formula X-A-X', wherein X and X' are independently hydroxy, or a leaving group, such as halogen, acyloxy, or alkyl or aryl sulfonyloxy, for the preparation of a compound of the formula (II)

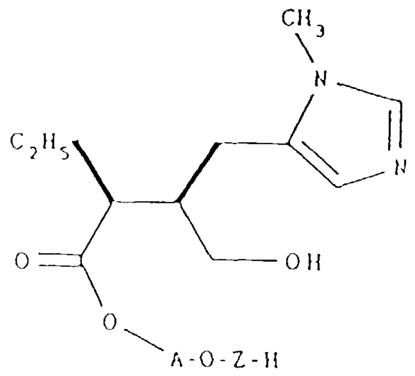

(II)

wherein Z is

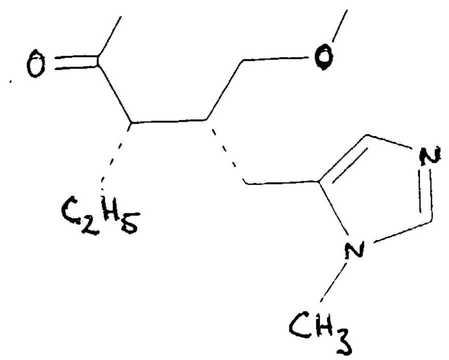

which, if desired, is converted to a compound of the formula I', wherein Y is different from hydrogen, by reacting the same with an acid of the formula $RCO_2H$, and when R is different from R', thereafter with an acid of the formula $R'CO_2H$, or with a functional derivative of these acids, and optionally the compound of the formula I' is converted to its acid addition salt, or

b) for the preparation of a compound of the formula I", a compound of the formula III or III' respectively

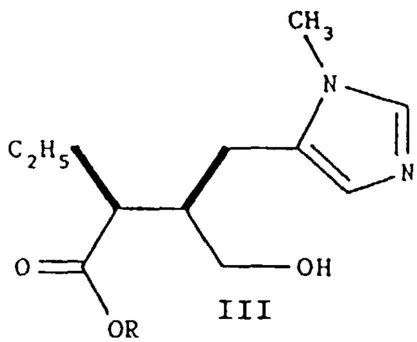

III and/or

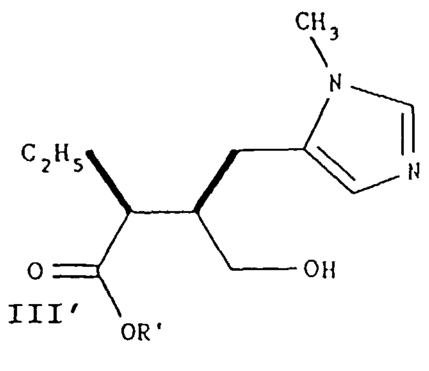

III'

wherein R and R' have the meaning given above, is reacted with a dicarboxylic acid of the formula IV

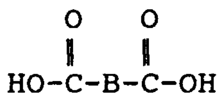

(IV)

wherein B has the meaning given above, or with a bifunctional acid derivative thereof, and optionally the compound obtained with the formula I'' is converted to its acid addition salt, or

c) for the preparation of a compound of the formula I''', a compound of the formula

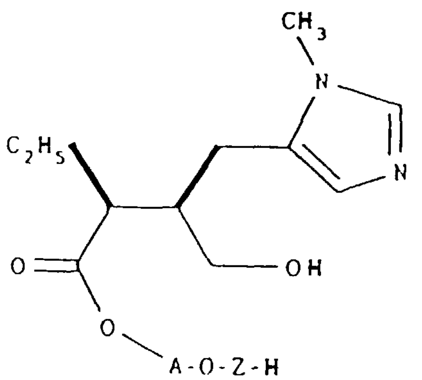

(II)

wherein A and Z have the meanings given above, is reacted with a dicarboxylic acid of the formula IV

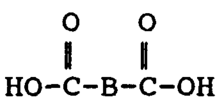

(IV)

wherein B has the meaning given above, or with a bifunctional acid derivative thereof, and optionally the obtained compound with the formula I''' is converted to its acid addition salt.

**6.** Pharmaceutical composition **characterized** in that it as the active agent contains a compound according to the formula I', I'' or I''' in Claim 1.

## Patentansprüche

**1.** Bispilocarpinsäureesterderivate der Formeln I', I" und I'"

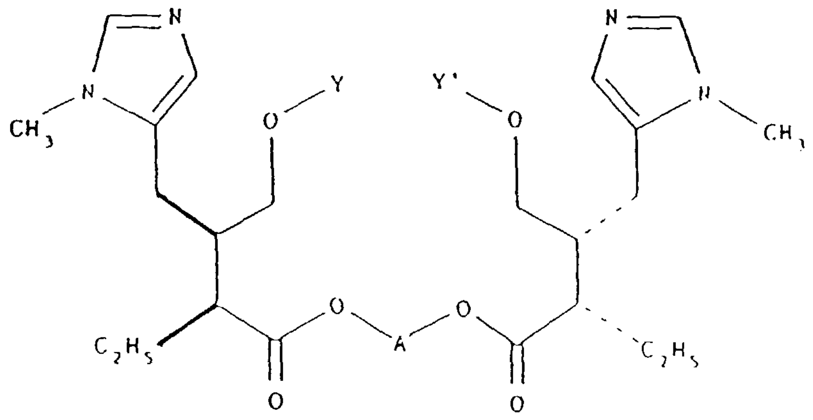

(I')

worin

Y Wasserstoff oder

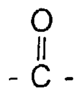

R bedeutet, worin R Wasserstoff, $C_1$-$C_{18}$-Alkyl, $C_2$-$C_{18}$-Alkenyl, $C_2$-$C_{18}$-Alkynyl, unsubstituiertes oder durch $C_1$-$C_4$-Alkyl substituiertes $C_3$-$C_7$-Cycloalkyl oder $C_3$-$C_7$-Cycloalkenyl, unsubstituiertes oder durch $C_1$-$C_4$-Alkyl, $C_1$-$C_4$-Alkoxy, Nitro oder Halogen substituiertes Aryl oder Arylniederalkyl mit bis zu 6 Kohlenstoffen im Alkylteil bedeutet, und

Y' Wasserstoff oder die Gruppe

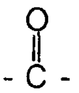

R' bedeutet, worin R' die gleiche Bedeutung wie R oben hat, wobei R' gleich wie R oder verschieden von R sein kann, A gegebenenfalls durch Hydroxy oder durch geschütztes Hydroxy substituiertes $C_1$-$C_{18}$-Alkylen, $C_2$-$C_{18}$-Alkenylen oder $C_2$-$C_{18}$-Alkynylen, die durch unsubstituiertes oder durch $C_1$-$C_4$-Alkyl substituiertes $C_3$-$C_7$-Cycloalkyl, $C_3$-$C_7$-Cycloalkenyl, unsubstituiertes oder durch $C_1$-$C_4$-Alkyl, $C_1$-$C_4$-Alkoxy, Nitro oder Halogen substituiertes Aryl oder Arylniederalkyl mit bis zu 6 Kohlenstoffen im Alkylteil substituiert sein können, bedeutet, oder A unsubstituiertes oder durch $C_1$-$C_4$-Alkyl substituiertes $C_3$-$C_7$-Cycloalkylen oder $C_3$-$C_7$-Cycloalkenylen, oder unsubstituiertes oder durch $C_1$-$C_4$-Alkyl, $C_1$-$C_4$-Alkoxy, Nitro oder Halogen substituiertes Arylen bedeutet, oder A das obengenannte Alkylen, Alkenylen oder Alkynylen bedeutet, das als ein Kettenglied das oben definierte Cycloalkylen, Cycloalkenylen oder Arylengruppe enthält,

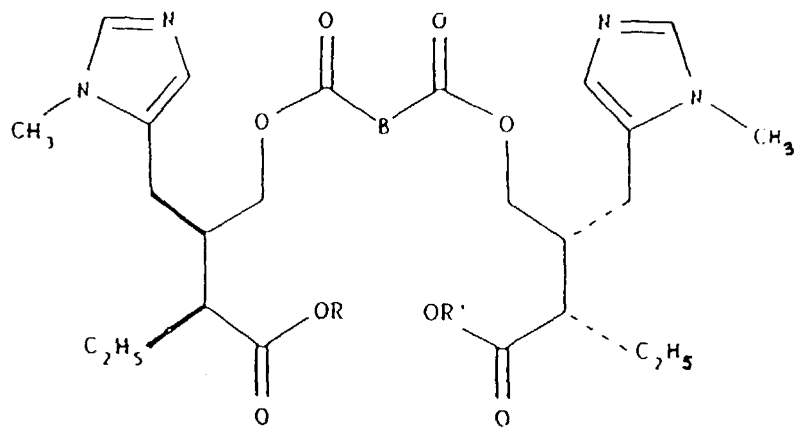

(I")

worin R die oben angegebenen Bedeutungen hat,

R' die oben angegebenen Bedeutung hat, und B die oben für A angegebenen Bedeutungen hat, und

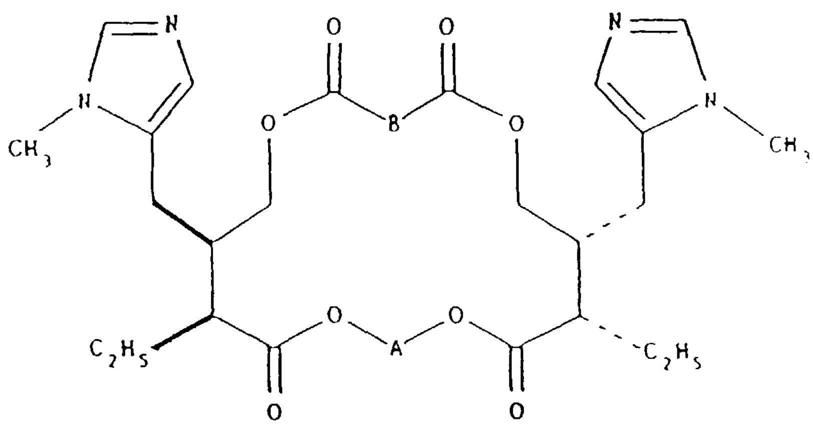

(I"')

worin A und B die oben angegebenen Bedeutungen haben, sowie die Säureadditionssalze der genannten Verbindungen.

2. Bispilocarpinderivat nach Anspruch 1, dadurch **gekennzeichnet**, dass dieses ein (Xylylen)bispilocarpat, insbesondere (1,4-Xylylen)bispilocarpat, ist.

3. Bispilocarpinderivat nach Anspruch 1, dadurch **gekennzeichnet**, dass dieses O,O'-Dipropionyl-, O,O'-Dibutyryl- oder O,O'-Dicyclopropylcarbonyl-(1,4-xylylen)bispilocarpat ist.

4. Bispilocarpinderivat nach Anspruch 1, dadurch **gekennzeichnet**, dass dieses ein (Dibenzyl)bispilocarpat, insbesonde O,O'-Glytaryl(dibenzyl)bispilocarpat ist.

5. Verfahren zur Herstellung der Verbindungen der Formeln I', I" und I"' in Anspruch 1, dadurch **gekennzeichnet**, dass

a) zur Herstellung einer Verbindung der Formel I', Pilocarpinsäure der Formel

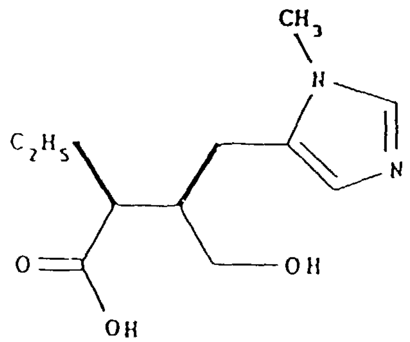

oder deren Salz mit einer Verbindung der Formel X-A-X', worin X und X' unabhängig voneinander Hydroxy, oder eine abgehende Gruppe, wie Halogen, Acyloxy oder Alkyl- oder Arylsulfonyloxy bedeuten, umgesetzt wird, zur Herstellung einer Verbindung der Formel (II)

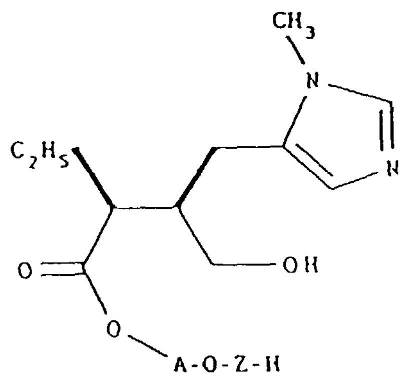

(II)

worin Z

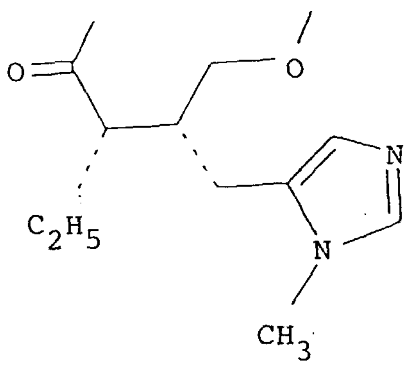

ist, welche Verbindung, wenn erwünscht, in eine Verbindung der Formel I' umgewandelt wird, worin Y von Wasserstoff verschieden ist, durch Umsetzung derselben mit einer Säure der Formel $RCO_2H$, und wenn R von R' verschieden ist, danach mit einer Säure der Formel $R'CO_2$ H, oder mit einem funktionellen Derivat dieser Säuren, und gegebenenfalls die Verbindung der Formel I' in deren Säureadditionssalz umgewandelt wird, oder

b) zur Herstellung einer Verbindung der Formel I'', eine Verbindung der Formel III bzw. der Formel III'

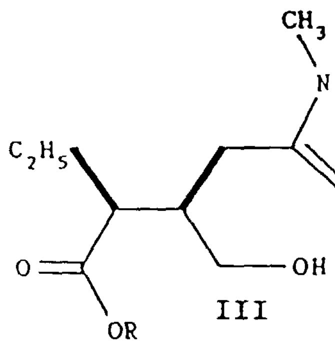

III

und/oder

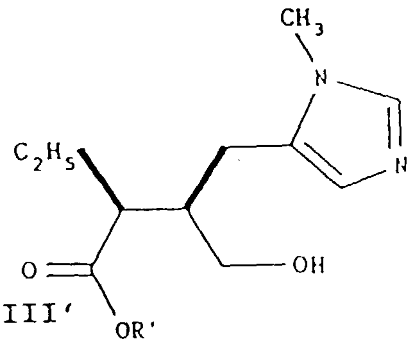

III'

worin R und R' die oben angegebenen Bedeutungen haben, mit einer Dicarbonsäure der Formel IV

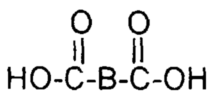

(IV)

worin B die oben angegebenen Bedeutungen hat, oder mit deren bifunktionellen Säurederivat umgesetzt wird, und gegebenenfalls die erhaltene Verbindung der Formel I'' in deren Säureadditionssalz umgewandelt wird, oder

c) zur Herstellung einer Verbindung der Formel I''', eine Verbindung der Formel

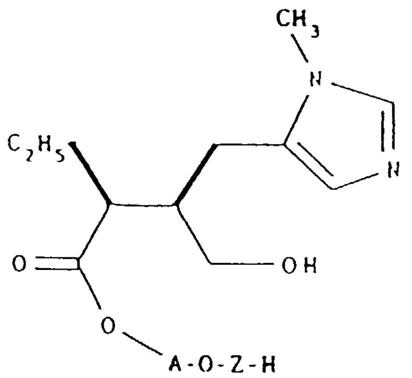

(II)

worin A und Z die oben angegebenen Bedeutungen haben, mit einer Dicarbonsäure der Formel IV

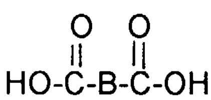

(IV)

worin B die oben angegebenen Bedeutungen hat, oder mit ihrem bifunktionellen Säurederivat umgesetzt wird, und gegebenenfalls die erhaltene Verbindung der Formel I''' in deren Säureadditionssalz umgewandelt wird.

6. Pharmazeutische Zusammensetzung, dadurch **gekennzeichnet**, dass sie als den Wirkstoff eine Verbindung der Formel I', I'' oder I''' in Anspruch 1 enthält.

## Revendications

**1.** Dérivés du type ester d'acide bispilocarpique de formules I', I" et I'"

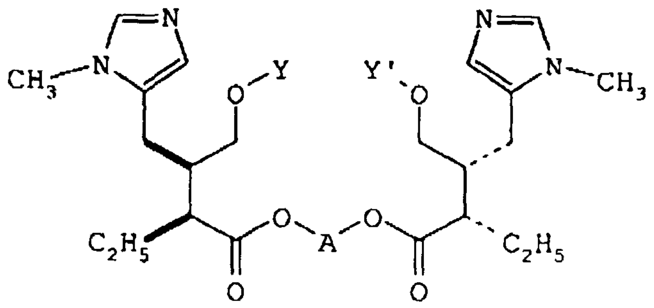

(I')

dans laquelle Y est H ou -CO-R où R est H, un groupe alkyle en $C_1$-$C_{18}$, alkényle en $C_2$-$C_{18}$ ou alkynyle on $C_2$-$C_{18}$, un groupe cycloalkyle en $C_3$-$C_7$ ou cycloalkényle on $C_3$-$C_7$ qui est non substitué ou alkyl(en $C_1$-$C_4$)-substitué, un groupe aryle ou aryl-(alkyle inférieur) ayant jusqu'à 6 atomes de carbone dans le fragment alkyle qui est non substitué ou substitué par alkyle en $C_1$-$C_4$, alkoxy en $C_1$-$C_4$, nitro ou halogène, et Y' représente H ou un groupe -CO-R', vu R' à la même signification que R ci-dessus, R' étant identique différent de R, A est un groupe alkylène en $C_1$-$C_{18}$, alkénylène on $C_2$-$C_{18}$ ou alkynylène on $C_2$-$C_{18}$ qui est le cas échéant OH-substitué ou (OH protégé)-substitué, et qui peut être substitué par des groupements cycloalkyle en $C_3$-$C_7$ ou cycloalkényle on $C_3$-$C_7$, qui sont non substitués ou alkyl(en $C_1$-$C_4$)-substitués, aryle ou aryl-(alkyle inférieur) ayant jusqu'à 6 atomes de carbone dans le fragment alkyle, qui sont non substitués ou substitués par alkyle en $C_1$-$C_4$, alkoxy on $C_1$-$C_4$, nitro ou halogène, ou A est un groupe cycloalkylène on $C_3$-$C_7$ ou cycloalkénylène on $C_3$-$C_7$ non substitué ou alkyl (en $C_3$-$C_4$)-substitué, ou arylène non substitué ou substitué par alkyle on $C_1$-$C_4$, alkoxy on $C_1$-$C_4$, nitro ou halogène, ou A est ledit groupe alkylène, alkénylène ou alkynylène précité, qui en tant qu'élément caténaire contient ledit groupe cycloalkylène, cycloalkénylène ou arylène défini ci-dessus,

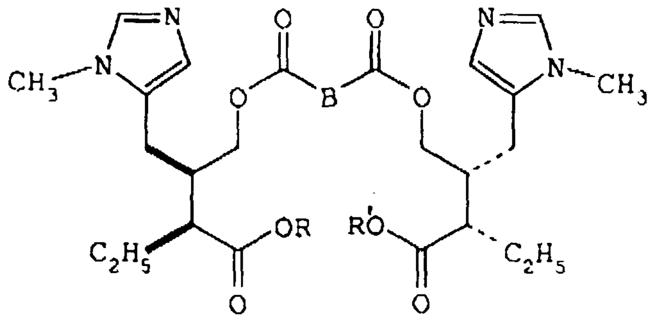

(I")

dans laquelle R est défini comme indiqué ci-dessus, R' est défini comme indiqué ci-dessus, et B reprend la définition de A ci-dessus,

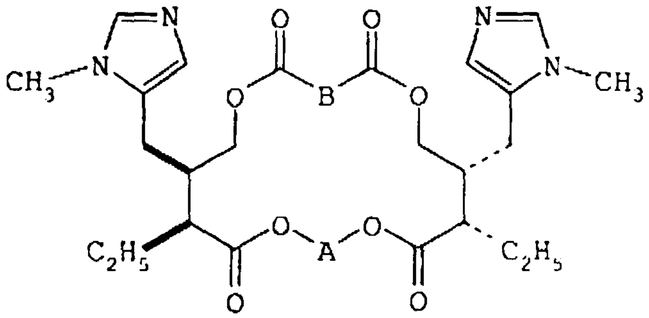

(I"')

dans laquelle A et B sont définis comme indiqué ci-dessus, ainsi que leurs sels d'addition d'acide.

**2.** Dérivé de bispilocarpine suivant la revendication 1, caractérisé en ce qu'il s'agit d'un (xylylène) bispilocarpate, notamment un (1,4-xylylène) bispilocarpate.

**3.** Dérivé de bispilocarpine suivant la revendication 1, caractérisé en ce qu'il s'agit de O,O'-dipropionyl, O,O'-dibutyryl, ou O,O'-dicyclopropylcarbonyl (1,4-xylylène) bispilocarpate.

**4.** Dérivé de bispilocarpine suivant la revendication 1, caractérisé en ce qu'il s'agit d'un (dibenzyl) bispilocarpate, notamment le O,O'-glutaryl (dibenzyl) bispilocarpate.

**5.** Procédé pour la préparation de composés de formules I', I" et I"' selon la revendication 1, caractérisé en ce que

a) pour la préparation d'un composé de formule I', on fait réagir un acide de formule

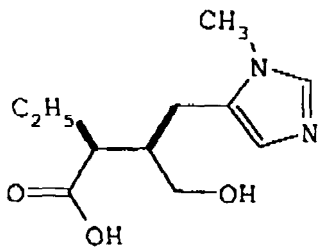

ou l'un de ses sels, avec un composé de formule X-A-X', où X et X' sont indépendamment OH, un groupe clivable, tel que halogène, acyloxy, ou alkyl- ou arylsulfonyloxy, pour la préparation d'un composé de formule (II)

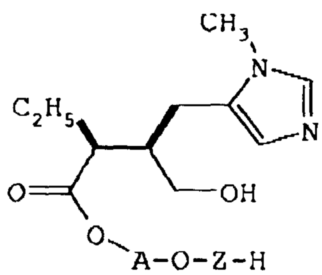

(II)

où Z est

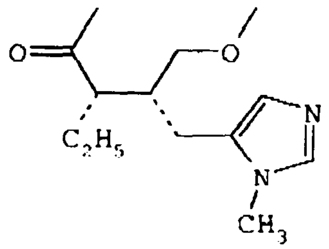

qui, si nécessaire, est transformé en un composé de formule I', dans lequel Y est différent de H par réaction dudit composé de formule II avec un acide de formule $RCO_2H$, et, quand R est différent de R', par réaction ultérieure avec un acide de formule $R'CO_2H$, ou avec un dérivé fonctionnel de ces acides, et le cas échéant le composé de formule I' est transformé en son sel d'addition d'acide, ou

b) pour la préparation d'un composé de formule I'', on fait réagir un composé de formule III ou respectivement III'

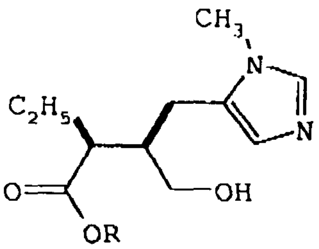

(III)

et/ou

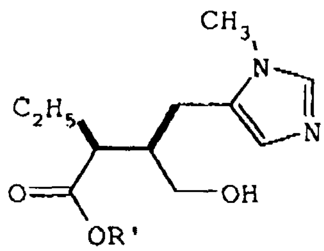

(III')

dans lesquels R et R' sont définis comme indiqué ci-dessus, avec un acide dicarboxylique de formule IV

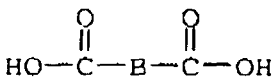

(IV)

où B est défini comme indiqué ci-dessus, ou avec un dérivé d'acide bifonctionnel, et le cas échéant le produit de formule I'' ainsi obtenu est transformé en son sel d'addition d'acide, ou

c) pour la préparation d'un composé de formule I''', on fait réagir un composé de formule

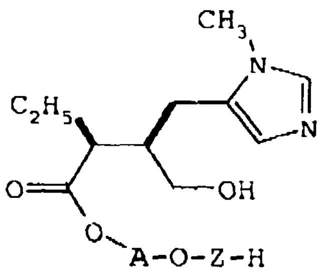

(II)

où A et Z sont définis comme indiqué ci-dessus, avec un acide dicarboxylique de formule IV

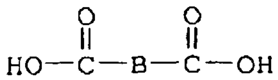

(IV)

où B est défini comme indiqué ci-dessus, ou avec son dérivé d'acide bifonctionnel, et le cas échéant le composé dc formule I''' ainsi obtenu est transformé en son sel d'addition d'acide.

**6.** Composition pharmaceutique caractérisée en ce qu'elle contient en tant qu'ingrédient actif un composé de formule I', I'' ou I''' selon la revendication 1.